# EUROPEAN PATENT SPECIFICATION

(11) **EP 1 471 853 B1**
(45) Date of publication and mention of the grant of the patent: **22.03.2017**
(21) Application number: 03715988.6
(22) Date of filing: 06.02.2003
(51) Int. Cl.: A61F 2/06

(54) **MEDICAL DEVICE WITH COATING THAT PROMOTES ENDOTHELIAL CELL ADHERENCE AND DIFFERENTIATION**
MEDIZINISCHE VORRICHTUNG MIT EINEM ÜBERZUG ZUR FÖRDERUNG DER ANHAFTUNG UND DIFFERENZIERUNG VON ENDOTHELZELLEN
DISPOSITIF MEDICAL RECOUVERT D'UN REVETEMENT QUI FACILITE LA FIXATION ET LA DIFFERENCIATION DE CELLULES ENDOTHELIALES

(30) Priority: 06.02.2002 US 354680 P
(43) Date of publication of application: 03.11.2004
(60) Divisional application: 12184398.1
(73) Proprietor: OrbusNeich Medical, Inc., Ft Lauderdale, FL 33309 (US)
(72) Inventor: KUTRYK, Michael J.B., Toronto, Ontario M4S 1J7 (CA); COTTONE, Robert, J., Jr., Davie, FL 33330 (US); ROWLAND, Stephen, M., Miami, FL 33157 (US); KULISZEWSKI, Michael, A., Mississauga, Ontario L5R 1Y2 (CA)
(74) Representative: Jilderda, Anne Ayolt
(86) International application number: PCT/US2003/003645
(87) International publication number: WO 2003/065881

(56) References cited:
- WO-A1-01/68158
- DE-A1- 19 903 385
- US-A- 5 310 669
- US-A- 5 338 571
- DEKKER A. ET AL.: 'Improved adhesion and proliferation of human endothelial cells on polyethylene precoated with monoclonal antibodies directed against cell membrane antigens and extracellular matrix proteins' THROMBOSIS AND HAEMOSTASIS vol. 66, no. 6, 1991, pages 715 - 724, XP009038150
- ASAHARA T. ET AL.: 'Isolation of putative progenitor endothelial cells for angiogenesis' SCIENCE vol. 275, 14 February 1997, pages 964 - 967, XP002059361
- STEVEN O. MARX ET AL.: "Vascular Smooth Muscle Cell Proliferation in Restenosis", CIRC. CARDIOVASC. INTERV., 1 February 2011 (2011-02-01), pages 104-111, DOI: 10.1161/CIRCINTERVENTIONS.110.957332
- NEIL SWANSON ET AL.: "Vascular Endothelial Growth Factor (VEGF)-Eluting Stents: In vivo Effects on Thrombosis, Endotheliazation and Intimal Hyperplasia", J. INVASIVE CARDIOL., vol. 15, no. 12, 1 December 2003 (2003-12-01), pages 688-692,

## Description

### FIELD OF INVENTION

The present invention relates to the field of medical devices implanted in vessels or hollowed organs within the body. In particularly, the present invention relates to artificial, intraluminal blood contacting surfaces of medical devices such as coated stents, stent grafts, synthetic vascular grafts, heart valves, catheters and vascular prosthetic filters. The coating on the implanted medical device promotes progenitor endothelial cells to adhere, grow and differentiate on the surface of the implanted device to form a functional endothelium, and thereby inhibiting intimal hyperplasia of the blood vessel or organ at the site of the implant.

### BACKGROUND OF INVENTION

Atherosclerosis is one of the leading causes of death and disability in the world. Atherosclerosis involves the deposition of fatty plaques on the lumenal surface of arteries. This deposition of fatty plaques causes narrowing of the cross-sectional area of the artery. Ultimately, this deposition blocks blood flow distal to the lesion causing ischemic damage to the tissues supplied by the artery.

Coronary arteries supply the heart with blood. Coronary artery atherosclerosis disease (CAD) is the most common, serious, chronic, life-threatening illness in the United States, affecting more than 11 million persons. The social and economic costs of coronary atherosclerosis vastly exceed those of most other diseases. Narrowing of the coronary artery lumen causes destruction of heart muscle resulting first in angina, followed by myocardial infarction and finally death. There are over 1.5 million myocardial infarctions in the United States each year. Six hundred thousand (or 40%) of those patients suffer an acute myocardial infarction and more than three hundred thousand of those patients die before reaching the hospital. (Harrison's Principles of Internal Medicine, 14th Edition, 1998).

CAD can be treated using percutaneous translumenal coronary balloon angioplasty (PTCA). More than 400,000 PTCA procedures are performed each year in the United States. In PTCA, a balloon catheter is inserted into a peripheral artery and threaded through the arterial system into the blocked coronary artery. The balloon is then inflated, the artery stretched, and the obstructing fatty plaque flattened, thereby increasing the cross-sectional flow of blood through the affected artery. The therapy, however, does not usually result in a permanent opening of the affected coronary artery. As many as 50% of the patients who are treated by PTCA require a repeat procedure within six months to correct a re-narrowing of the coronary artery. Medically, this re-narrowing of the artery after treatment by PTCA is called restenosis. Acutely, restenosis involves recoil and shrinkage of the vessel. Subsequently, recoil and shrinkage of the vessel are followed by proliferation of medial smooth muscle cells in response to injury of the artery from PTCA. In part, proliferation of smooth muscle cells is mediated by release of various inflammatory factors from the injured area including thromboxane A₂, platelet derived growth factor (PDGF) and fibroblast growth factor (FGF). A number of different techniques have been used to overcome the problem of restenosis, including treatment of patients with various pharmacological agents or mechanically holding the artery open with a stent. (Harrison's Principles of Internal Medicine, 14th Edition, 1998).

Of the various procedures used to overcome restenosis, stents have proven to be the most effective. Stents are metal scaffolds that are positioned in the diseased vessel segment to create a normal vessel lumen. Placement of the stent in the affected arterial segment prevents recoil and subsequent closing of the artery. Stents can also prevent local dissection of the artery along the medial layer of the artery. By maintaining a larger lumen than that created using PTCA alone, stents reduce restenosis by as much as 30%. Despite their success, stents have not eliminated restenosis entirely. (Suryapranata et al. 1998. Randomized comparison of coronary stenting with balloon angioplasty in selected patients with acute myocardial infarction. Circulation 97:2502-2502).

Narrowing of the arteries can occur in vessels other than the coronary arteries, including the aortoiliac, infrainguinal, distal profunda femoris, distal popliteal, tibial, subclavian and mesenteric arteries. The prevalence of peripheral artery atherosclerosis disease (PAD) depends on the particular anatomic site affected as well as the criteria used for diagnosis of the occlusion. Traditionally, physicians have used the test of intermittent claudication to determine whether PAD is present. However, this measure may vastly underestimate the actual incidence of the disease in the population. Rates of PAD appear to vary with age, with an increasing incidence of PAD in older individuals. Data from the National Hospital Discharge Survey estimate that every year, 55,000 men and 44,000 women had a first-listed diagnosis of chronic PAD and 60,000 men and 50,000 women had a first-listed diagnosis of acute PAD. Ninety-one percent of the acute PAD cases involved the lower extremity. The prevalence of comorbid CAD in patients with PAD can exceed 50%. In addition, there is an increased prevalence of cerebrovascular disease among patients with PAD.

PAD can be treated using percutaneous translumenal balloon angioplasty (PTA). The use of stents in conjunction with PTA decreases the incidence of restenosis. However, the post-operative results obtained with medical devices such as stents do not match the results obtained using standard operative revascularization procedures, i.e., those using a venous or prosthetic bypass material. (Principles of Surgery, Schwartz et al. eds., Chapter 20, Arterial Disease, 7th Edition, McGraw-Hill Health Professions Division, New York 1999).

Preferably, PAD is treated using bypass procedures where the blocked section of the artery is bypassed using a graft. (Principles of Surgery, Schwartz et al. eds., Chapter 20, Arterial Disease, 7th Edition, McGraw-Hill Health Professions Division, New York 1999). The graft can consist of an autologous venous segment such as the saphenous vein or a synthetic graft such as one made of polyester, polytetrafluoroethylene (PTFE), or expanded polytetrafluoroethylene (ePTFE), or other polymeric materials. The post-operative patency rates depend on a number of different factors, including the lumenal dimensions of the bypass graft, the type of synthetic material used for the graft and the site of outflow. Excessive intimal hyperplasia and thrombosis, however, remain significant problems even with the use of bypass grafts. For example, the patency of infrainguinal bypass procedures at 3 years using an ePTFE bypass graft is 54% for a femoral-popliteal bypass and only 12% for a femoral-tibial bypass.
WO01/68158 A discloses the coating of a stent or synthetic graft with a matrix incorporating antibodies that stimulate adherence of circulating progenitor endothelial cells to form an endothelial cell layer on the surface of the medical device.

Consequently, there is a significant need to improve the performance of stents, synthetic bypass grafts, and other chronic blood contacting surfaces and or devices, in order to further reduce the morbidity and mortality of CAD and PAD.

With stents, the approach has been to coat the stents with various antithrombotic or anti-restenotic agents in order to reduce thrombosis and restenosis. For example, impregnating stents with radioactive material appears to inhibit restenosis by inhibiting migration and proliferation of myofibroblasts. (U.S. Patent Nos. 5,059,166, 5,199,939 and 5,302,168). Irradiation of the treated vessel can cause severe edge restenosis problems for the patient. In addition, irradiation does not permit uniform treatment of the affected vessel.

Alternatively, stents have also been coated with chemical agents such as heparin, phosphorylcholine, rapamycin, and taxol, all of which appear to decrease thrombosis and/or restenosis. Although heparin and phosphorylcholine appear to markedly reduce thrombosis in animal models in the short term, treatment with these agents appears to have no long-term effect on preventing restenosis. Additionally, heparin can induce thrombocytopenia, leading to severe thromboembolic complications such as stroke. Therefore, it is not feasible to load stents with sufficient therapeutically effective quantities of either heparin or phosphorylcholine to make treatment of restenosis in this manner practical.

Synthetic grafts have been treated in a variety of ways to reduce postoperative restenosis and thrombosis. (Bos et al. 1998. Small-Diameter Vascular Graft Prostheses:Current Status Archives Physio. Biochem. 106:100-115). For example, composites of polyurethane such as meshed polycarbonate urethane have been reported to reduce restenosis as compared with ePTFE grafts. The surface of the graft has also been modified using radiofrequency glow discharge to fluorinate the polyterephthalate graft. Synthetic grafts have also been impregnated with biomolecules such as collagen. However, none of these approaches has significantly reduced the incidence of thrombosis or restenosis over an extended period of time.

The endothelial cell (EC) layer is a crucial component of the normal vascular wall, providing an interface between the bloodstream and the surrounding tissue of the blood vessel wall. Endothelial cells are also involved in physiological events including angiogenesis, inflammation and the prevention of thrombosis (Rodgers GM. FASEB J 1988;2:116-123.). In addition to the endothelial cells that compose the vasculature, recent studies have revealed that ECs and endothelial progenitor cells (EPCs) circulate postnatally in the peripheral blood (Asahara T, et al. Science 1997;275:964-7; Yin AH, et al. Blood 1997;90:5002-5012; Shi Q, et al. Blood 1998;92:362-367; Gehling UM, et al. Blood 2000;95:3106-3112; Lin Y, et al. J Clin Invest 2000;105:71-77). EPCs are believed to migrate to regions of the circulatory system with an injured endothelial lining, including sites of traumatic and ischemic injury (Takahashi T, et al. Nat Med 1999;5:434-438). In normal adults, the concentration of EPCs in peripheral blood is 3-10 cells/mm³ (Takahashi T, et al. Nat Med 1999;5:434-438; Kalka C, et al. Ann Thorac Surg. 2000;70:829-834). It is now evident that each phase of the vascular response to injury is influenced (if not controlled) by the endothelium. It is believed that the rapid reestablishment of a functional endothelial layer on damaged stented vascular segments may help to prevent these potentially serious complications by providing a barrier to circulating cytokines, peventing adverse effects of a thrombus, and by the ability of endothelial cells to produce substances that passivate the underlying smooth muscle cell layer. (Van Belle et al. 1997. Stent Endothelialization. Circulation 95:438-448; Bos et al. 1998. Small-Diameter Vascular Graft Prostheses:Current Status Archives Physio. Biochem. 106:100-115).

Endothelial cells have been encouraged to grow on the surface of stents by local delivery of vascular endothelial growth factor (VEGF), an endothelial cell mitogen, after implantation of the stent (Van Belle et al. 1997. Stent Endothelialization. Circulation 95:438-448.). While the application of a recombinant protein growth factor, VEGF in saline solution at the site of injury induces desirable effects, the VEGF is delivered to the site of injury after stent implantation using a channel balloon catheter. This technique is not desirable since it has demonstrated that the efficiency of a single dose delivery is low and produces inconsistent results. Therefore, this procedure cannot be reproduced accurately every time.

Synthetic grafts have also been seeded with endothelial cells, but the clinical results with endothelial seeding have been generally poor, i.e., low post-operative patency rates (Lio et al. 1998. New concepts and Materials in Microvascular Grafting: Prosthetic Graft Endothelial Cell Seeding and Gene Therapy. Microsurgery 18:263-256) due most likely to the fact the cells did not adhere properly to the graft and/or lost their EC function due to *ex-vivo* manipulation.

Endothelial cell growth factors and environmental conditions *in situ* are therefore essential in modulating endothelial cell adherence, growth and differentiation at the site of blood vessel injury. Accordingly, there is a need for the development of new methods and compositions for coating medical devices, including stents and synthetic grafts, which would promote and accelerate the formation of a functional endothelium on the surface of implanted devices so that a confluent EC monolayer is formed on the target blood vessel segment or grafted lumen and inhibiting neo-intimal hyperplasia. This type of coating will not only inhibit restenosis, but also will inhibit thromboembolic complications resulting from implantation of the device. Methods and compositions that provide such improvement will eliminate the drawbacks of previous technology and have a significant positive impact on the morbidity and mortality associated with CAD and PAD.

### SUMMARY OF INVENTION

It is an object of the invention to provide coated medical devices such as stents, stent grafts, heart valves, catheters, vascular prosthetic filters, artificial heart, external and internal left ventricular assist devices (LVADs), and synthetic vascular grafts, for the treatment of vascular diseases, including restenosis, artherosclerosis, thrombosis, blood vessel obstruction, and the like. The coating on the present medical device comprises a biocompatible matrix, at least one type of substance incorporated into the matrix and selected from the group consisting of antibodies, antibody fragments, combinations thereof and small molecules, and at least one growth factor, for modulating adherence, growth and differentiation of captured progenitor endothelial cells on the surface of the medical device to induce the formation of a functional endothelium to inhibit intimal hyperplasia in preventing restenosis, thereby improving the prognosis of patients being treated with vascular disease.

In one embodiment, the biocompatible matrix comprises an outer surface for attaching a therapeutically effective amount of at least one type of antibody, antibody fragment, or a combination of the antibody and the antibody fragment. The present antibody or antibody fragment recognizes and binds an antigen on a the cell membrane or surface of progenitor endothelial cells so that the cell is immobilized on the surface of the matrix. Additionally, the coating comprises a therapeutically effective amount of at least one compound for stimulating the immobilized progenitor endothelial cells to accelerate the formation of a mature, functional endothelium on the surface of the medical device.

The medical device of the invention can be any device used for implanting into an organ or body part comprising a lumen, and can be, but is not limited to, a stent, a stent graft, a synthetic vascular graft, a heart valve, a catheter, a vascular prosthetic filter, a pacemaker, a pacemaker lead, a defibrilator, a patent foramen ovale (PFO) septal closure device, a vascular clip, a vascular aneurysm occluder, a hemodialysis graft, a hemodialysis catheter, an atrioventricular shunt, an aortic aneurysm graft device or components, a venous valve, a suture, a vascular anastomosis clip, an indwelling venous or arterial catheter, a vascular sheath and a drug delivery port. The medical device can be made of numerous materials depending on the device. For example, a stent of the invention can be made of stainless steel, Nitinol (NiTi), or chromium alloy. Synthetic vascular grafts can be made of a cross-linked PVA hydrogel, polytetrafluoroethylene (PTFE), expanded polytetrafluoroethylene (ePTFE), porous high density polyethylene (HDPE), polyurethane, and polyethylene terephthalate.

The biocompatible matrix forming the coating of the present device comprises a synthetic material such as polyurethanes, segmented polyurethane-urea/heparin, poly-L-lactic acid, cellulose ester, polyethylene glycol, polyvinyl acetate, dextran and gelatin, a naturally-occurring material such as basement membrane components such as collagen, elastin, laminin, fibronectin, vitronectin; heparin, fibrin, cellulose, and amorphous carbon, or fullerenes.

In an embodiment of the invention, the medical device comprises a biocompatible matrix comprising fullerenes. In this embodiment, the fullerene can range from about C₂₀ to about C₁₅₀ in the number of carbon atoms, and more particularly, the fullerene is C₆₀ or C₇₀. The fullerene of the invention can also be arranged as nanotubes on the surface of the medical device.

The antibody for providing to the coating of the medical device comprises at least one type of antibody or fragment of the antibody. The antibody can be a monoclonal antibody, a polyclonal antibody, a chimeric antibody, or a humanized antibody. The antibody or antibody fragment recognizes and binds a progenitor endothelial (endothelial cells, progenitor or stem cells with the capacity to become mature, functional endothelial cells) cell surface antigen and modulates the adherence of the cells onto the surface of the medical device. The antibody or antibody fragment of the invention can be covalently or noncovalently attached to the surface of the matrix, or tethered covalently by a linker molecule to the outermost layer of the matrix coating the medical device. In this aspect of the invention, for example, the monoclonal antibodies can further comprises Fab or F(ab')₂ fragments. The antibody fragment of the invention comprises any fragment size, such as large and small molecules which retain the characteristic to recognize and bind the target antigen as the antibody.

The antibody or antibody fragment of the invention recognize and bind antigens with specificity for the mammal being treated and their specificity is not dependent on cell lineage. In one embodiment, the antibody or fragment is specific for a human progenitor endothelial cell surface antigen such as CD133, CD34, CDw90, CD117, HLA-DR, VEGFR-1, VEGFR-2, Muc-18 (CD146), CD130, stem cell antigen (Sca-1), stem cell factor 1 (SCF/c-Kit ligand), Tie-2 and HAD-DR.

In another embodiment, the coating of the medical device comprises at least one layer of a biocompatible matrix as described above, the matrix comprising an outer surface for attaching a therapeutically effective amount of at least one type of small molecule of natural or synthetic origin. The small molecule recognizes and interacts with an antigen on a progenitor endothelial cell surface to immobilize the progenitor endothelial cell on the surface of the device to form an endothelium. The small molecules can be derived from a variety of sources such as cellular components such as fatty acids, proteins, nucleic acids, saccharides and the like and can interact with an antigen on the surface of a progenitor endothelial cell with the same results or effects as an antibody. In this aspect of the invention, the coating on the medical device can further comprise a compound such as a growth factor as described herewith in conjunction with the coating comprising an antibody or antibody fragment.

The growth factor of the coating of the invention comprises any compound which stimulates or accelerates the growth and differentiation of the progenitor cell into mature, functional endothelial cells. For example, a compound for use in the invention is a growth factor such as vascular endothelial growth factor (VEGF), basic fibroblast growth factor, platelet-induced growth factor, transforming growth factor beta 1, acidic fibroblast growth factor, osteonectin, angiopoietin 1 (Ang-1), angiopoietin 2 (Ang-2), insulin-like growth factor, granulocyte-macrophage colony-stimulating factor, platelet-derived growth factor AA, platelet-derived growth factor BB, platelet-derived growth factor AB and endothelial PAS protein 1. The invention also provides a medical device which has increased biocompatibility over prior art devices, and decreases or inhibits tissue-based excessive intimal hyperplasia and restenosis by decreasing or inhibiting smooth muscle cell migration, smooth muscle cell differentiation, and collagen deposition along the inner luminal surface at the site of implantation of the medical device.

In an embodiment of the invention, a method for coating a medical device comprises the steps of: applying at least one layer of a biocompatible matrix to the surface of the medical device, wherein the biocompatible matrix comprises at least one component selected from the group consisting of a polyurethane, a segmented polyurethane-urea/heparin, a poly-L-lactic acid, a cellulose ester, a polyethylene glycol, a polyvinyl acetate, a dextran, gelatin, collagen, elastin, laminin, fibronectin, vitronectin, heparin, fibrin, cellulose and carbon and fullerene, and
applying to the biocompatible matrix, simultaneously or sequentially, a therapeutically effective amounts of at least one type of antibody, antibody fragment or a combination thereof, and at least one compound which stimulates endothelial cell growth and differentiation.

### BRIEF DESCRIPTION OF DRAWINGS

FIG. 1A is a schematic representation of an antibody tethered covalently to the matrix by a cross-linking molecule. FIG. 1B shows a diagram of the C₆₀O molecule anchoring the matrix. FIG. 1C depicts a schematic representation of a stent coated with the matrix of the invention.

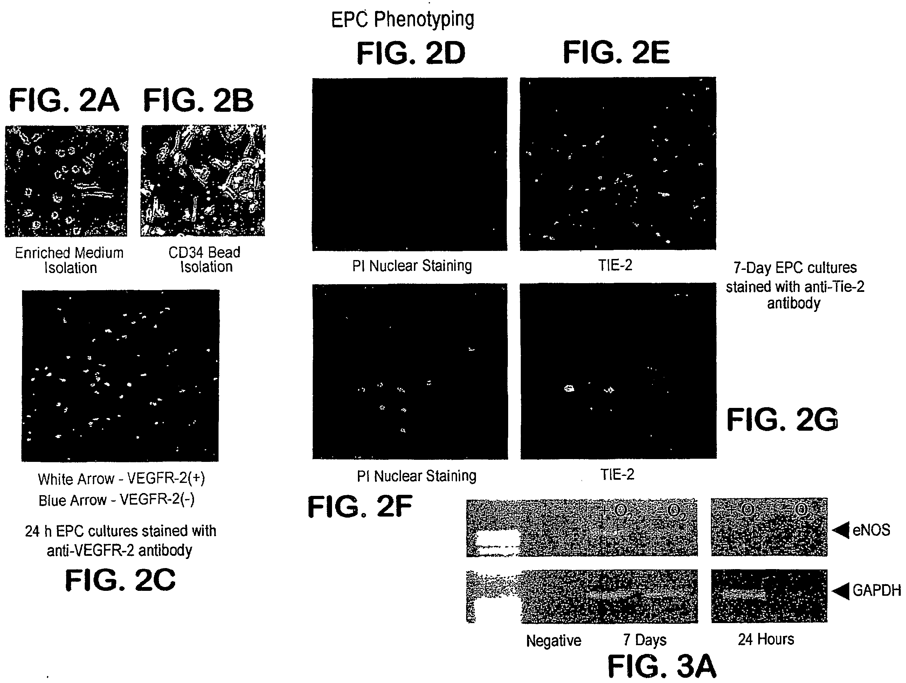

FIG. 2A is a phase contrast micrograph of progenitor endothelial cells adhered to a fibronectin-coated slide containing cells isolated by enriched medium. FIG. 2B is a phase contrast micrograph of progenitor endothelial cells adhered to a fibronectin-coated slide containing cells isolated by anti-CD34 antibody coated magnetic beads. FIGs. 2D and 2F are micrographs of the progenitor endothelial cells which had been incubated for 7 days and stained with PI nuclear stain. As seen in these figures, the cells express mature endothelial cell markers as shown by the antibody fluorescence for Tie-2 (FIGs. 2E and 2G) and VEGFR-2 (FIG. 2C) antibody reactivity.
FIGs. 3A and 3B are photographs of a 2% agarose gel stained with ethidium bromide of a semiquantitative RT-PCR for endothelial nitric oxide synthatase, eNOS and glyceraldehyde phosphate dehydrogenase, GAPDH. After 3 days (FIG. 3B) and 7 days (FIG. 3A) in culture on fibronectin-coated slides, the progenitor endothelial cells begin to express eNOS mRNA.
FIGs. 4A-4E are photomicrographs of HUVECs attached to the CMDx and anti-CD34 antibody (4A); gelatin and anti-CD34 antibody (4B); bare stainless steel disc (4C); CMDx coated and gelatin coated stainless steel disc which were incubated with HUVEC cell and stained with propidium iodide.
FIGs. 5A-5C are photomicrographs of a control, coated with CMDx without antibody. FIGs. 5D-5F are photomicrographs of control stainless steel discs coated with gelatin without antibody bound to its surface.
FIGs. 6A-6C are photomicrographs of stainless steel discs coated with CMDx matrix with anti-CD34 antibody bound to its surface. FIGs. 6D-6F are photomicrographs of stainless steel discs coated with gelatin matrix with antibody bound to its surface.
FIG. 7 is a photomicrograph of stainless steel discs coated with CMDx matrix with antibody bound to its surface, which was incubated with progenitor cells for 24 hours.
FIGs. 8A and 8B are photomicrographs of a stainless steel disc coated with CMDx matrix containing anti-CD34 antibody bound to its surface incubated with progenitor cells for 7 days and developed with anti-KDR antibodies.
FIGs. 9A and 9B photomicrograph of a stainless steel disc coated with CMDx matrix containing anti-CD34 antibody bound to its surface incubated with progenitor cells for 7days and developed with anti-Tie-2 antibodies.
FIGs. 10A-10C are phase contrast photomicrographs of stainless steel CMDx coated discs incubated with progenitor cells for 3 weeks in endothelial growth medium which show mature endothelial cells.
FIG. 11 is schematic diagram of a functional fullerene coated stent surface of the invention binding a progenitor cell.
FIGs. 12A-12D are photomicrographs of fullerene-coated samples without or with anti-CD34 antibody stained with Propidium bromide and anti-VEGFR-2 antibody.
13A-13D are photomicrographs of coronary artery explants which had been implanted for 4 weeks with a bare stainless steel stent (FIGs. 13A and 13C) and a fullerene-coated sample (FIGs.13B and 13D) taken at low and high magnification, respectively.

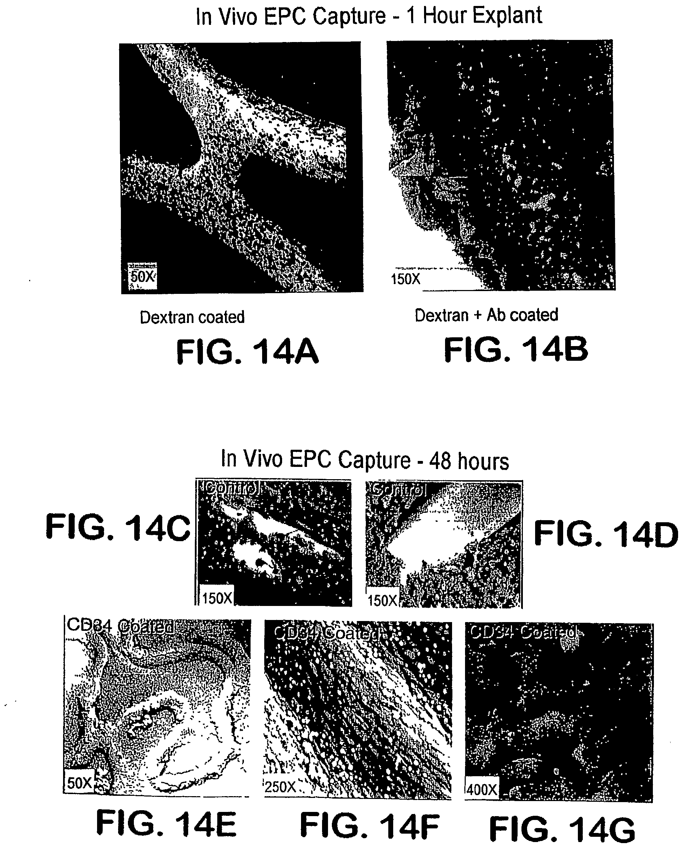

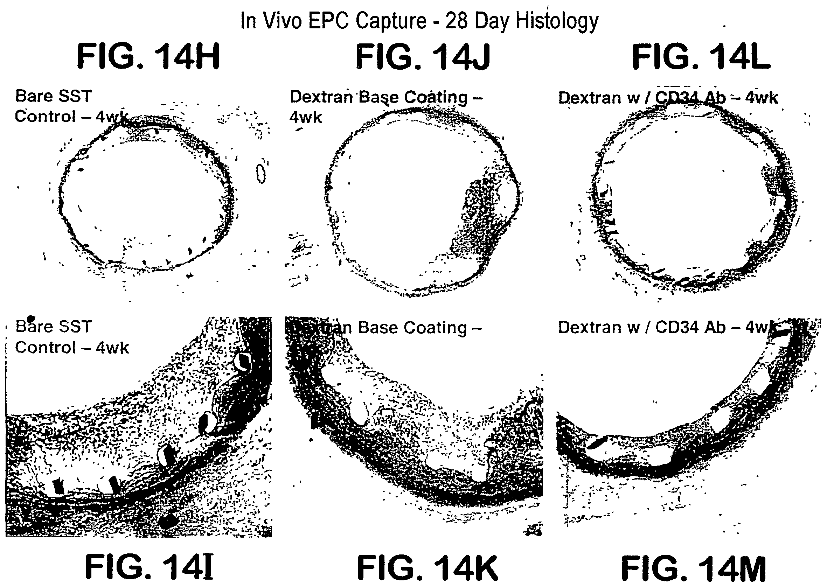

FIGs.14A -14G are scanning electron micrographs of 1 and 48 hours. Explants of dextran-coated (FIG. 14A) and dextran/anti-CD34 antibody-coated (14B) stents at 1 hour after implantation. FIGs. 14C and 14D show explants of control samples and FIGs. 14E-G are dextran/anti-CD34 antibody-coated stents at 48 hours after implantation. FIGs. 14H-14M are histological photomicrographs of cross-sections through coronary arteries of explants from male Yorkshire swine which were implanted for 4 weeks: uncoated (Bare stainless steel) (14H and 14I), dextran-coated control (14J and 14K), and dextran/anti-CD34 antibody-coated (14L and 14M).
FIGs. 15A, 15B and 15C are, respectively, confocal photomicrographs of 48 hours explants sections of a dextran-plasma-coated stent without antibody on its surface, and a dextran-plasma-coated/anti-CD34 antibody-coated stent of 18 mm in lenght.
FIGs. 16A and 16B are photomicrographs of a Propidium iodide and anti-lectin/FITC-conjugated sample.

### DETAILED DESCRIPTION

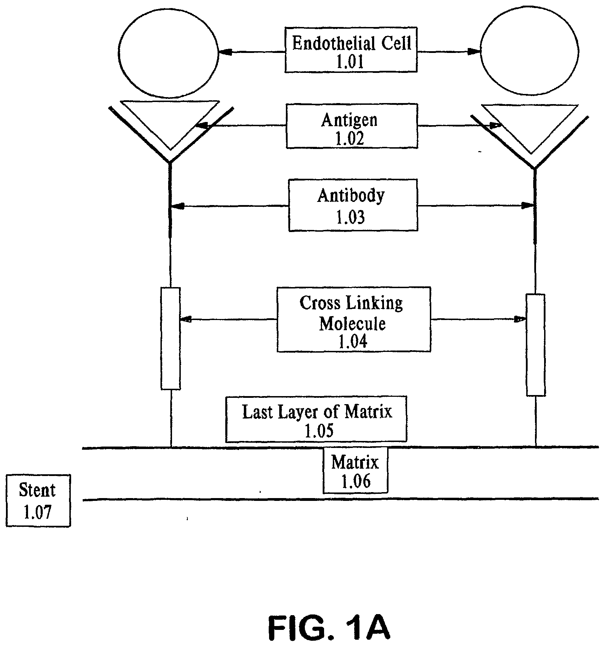

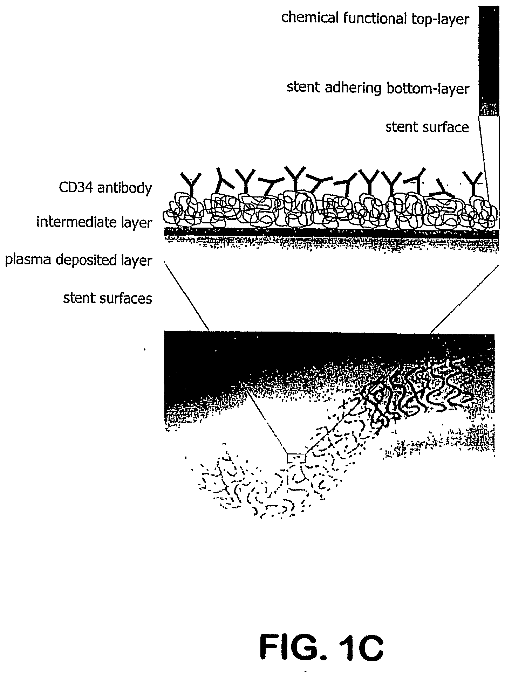

The present invention provides a coated, implantable medical device such as a stent, methods and compositions for coating the medical device, and methods of treating vascular disease with the coated medical device. FIGs. 1A-1C show a schematic representation of the surface coat of a medical device of the invention. The coat on the medical device comprises a biocompatible matrix for promoting the formation of a confluent layer of endothelial cells on the surface of the device to inhibit excessive intimal hyperplasia, and thereby preventing restenosis and thrombosis. In one embodiment, the matrix comprises a synthetic or naturally-occurring material in which a therapeutically effective amount of at least one type of antibody that promotes adherence of endothelial, progenitor or stem cells to the medical device, and at least one compound such as a growth factor, which stimulates endothelial cell growth and differentiation. Upon implantation of the device, the cells that adhere to the surface of the device transform into a mature, confluent, functional layer of endothelium on the luminal surface of the medical device. The presence of a confluent layer of endothelial cells on the medical device reduces the occurrence of restenosis and thrombosis at the site of implantation.

As used herein, "medical device" refers to a device that is introduced temporarily or permanently into a mammal for the prophylaxis or therapy of a medical condition. These devices include any that are introduced subcutaneously, percutaneously or surgically to rest within an organ, tissue or lumen of an organ, such as arteries, veins, ventricles or atrium of the heart. Medical devices may include stents, stent grafts, covered stents such as those covered with polytetrafluoroethylene (PTFE), expanded polytetrafluoroethylene (ePTFE), or synthetic vascular grafts, artificial heart valves, artificial hearts and fixtures to connect the prosthetic organ to the vascular circulation, venous valves, abdominal aortic aneurysm (AAA) grafts, inferior venal caval filters, permanent drug infusion catheters, embolic coils, embolic materials used in vascular embolization (e.g., cross-linked PVA hydrogel), vascular sutures, vascular anastomosis fixtures, transmyocardial revascularization stents and/or other conduits.

Coating of the medical device with the compositions and methods of this invention stimulates the development of a confluent endothelial cell layer on the surface of the medical device, thereby preventing restenosis as well as modulating the local chronic inflammatory response and other thromboembolic complications that result from implantation of the medical device.

The matrix coating the medical device can be composed of synthetic material, such as polymeric gel foams, such as hydrogels made from polyvinyl alcohol (PVA), polyurethane, poly-L-lactic acid, cellulose ester or polyethylene glycol. In one embodiment, very hydrophilic compounds such as dextran compounds can comprise the synthetic material for making the matrix. In another embodiment, the matrix is composed of naturally occurring materials, such as collagen, fibrin, elastin or amorphous carbon. The matrix may comprise several layers with a first layer being composed of synthetic or naturally occurring materials and a second layer composed of antibodies. The layers may be ordered sequentially, with the first layer directly in contact with the stent or synthetic graft surface and the second layer having one surface in contact with the first layer and the opposite surface in contact with the vessel lumen.

The matrix further comprises at least a growth factor, which stimulates endothelial cell proliferation and differentiation. For example, vascular endothelial cell growth factor (VEGF) and isoforms, basic fibroblast growth factor (bFGF), platelet-induced growth factor (PIGF), transforming growth factor beta 1 (TGF.*b*1), acidic fibroblast growth factor (aFGF), osteonectin, angiopoietin 1, angiopoietin 2, insulin-like growth factor (ILGF), platelet-derived growth factor AA (PDGF-AA), platelet-derived growth factor BB (PDGF-BB), platelet-derived growth factor AB (PDGF-AB), granulocyte-macrophage colony-stimulating factor (GM-CSF), and the like, or functional fragments thereof can be used in the invention.

In another embodiment, the matrix may comprise fullerenes, where the fullerenes range from about C₂₀ to about C₁₅₀ in carbon number. The fullerenes can also be arranged as nanotubes, that incorporate molecules or proteins. The fullerene matrix can also be applied to the surface of stainless steel, PTFE, or ePTFE medical devices, which layer is then functionalized and coated with antibodies and growth factor on its surface. Alternatively, the PTFE or ePTFE can be layered first on, for example, a stainless steel medical device followed by a second layer of fullerenes and then the antibodies and the growth factor are added.

The matrix may be noncovalently or covalently attached to the medical device. Antibodies and growth factors can be covalently attached to the matrix using hetero- or homobifunctional cross-linking reagents. The growth factor can be added to the matrix using standard techniques with the antibodies or after antibody binding.

As used herein, the term "antibody" refers to one type of monoclonal, polyclonal, humanized, or chimeric antibody or a combination thereof, wherein the monoclonal, polyclonal, humanized or chimeric antibody binds to one antigen or a functional equivalent of that antigen. The term antibody fragment encompasses any fragment of an antibody such as Fab, F(ab')₂, and can be of any size, i.e., large or small molecules, which have the same results or effects as the antibody. (An antibody encompasses a plurality of individual antibody molecules equal to 6.022 x 10²³ molecules per mole of antibody).

In an aspect of the invention, a stent or synthetic graft of the invention is coated with a biocompatible matrix comprising antibodies that modulate adherence of circulating progenitor endothelial cells to the medical device. The antibodies of the invention recognize and bind progenitor endothelial cells surface antigens in the circulating blood so that the cells are immobilized on the surface of the device. In one embodiment, the antibodies comprise monoclonal antibodies reactive (recognize and bind) with progenitor endothelial cell surface antigens, or a progenitor or stem cell surface antigen, such as vascular endothelial growth factor receptor-1, -2 and -3 (VEGFR-1, VEGFR-2 and VEGFR-3 and VEGFR receptor family isoforms), Tie-1, Tie2, CD34, Thy-1, Thy-2, Muc-18 (CD146), CD30, stem cell antigen-1 (Sca-1), stem cell factor (SCF or c-Kit ligand), CD133 antigen, VE-cadherin, P1H12, TEK, CD31, Ang-1, Ang-2, or an antigen expressed on the surface of progenitor endothelial cells. In one embodiment, a single type of antibody that reacts with one antigen can be used. Alternatively, a plurality of different antibodies directed against different progenitor endothelial cell surface antigens can be mixed together and added to the matrix. In another embodiment, a cocktail of monoclonal antibodies is used to increase the rate of epithelium formation by targeting specific cell surface antigens. In this aspect of the invention, for example, anti-CD34 and anti-CD133 are used in combination and attached to the surface of the matrix on a stent.

As used herein, a "therapeutically effective amount of the antibody" means the amount of an antibody that promotes adherence of endothelial, progenitor or stem cells to the medical device. The amount of an antibody needed to practice the invention varies with the nature of the antibody used. For example, the amount of an antibody used depends on the binding constant between the antibody and the antigen against which it reacts. It is well known to those of ordinary skill in the art how to determine therapeutically effective amounts of an antibody to use with a particular antigen.

As used herein, the term "compound" refers to any substance such as a growth factor such as one belonging to the angiopoietin family and VEGF family, and vitamins such as A and C, that stimulates the growth and differentiation of progenitor endothelial cells into mature, functional endothelial cells, which express molecules such as nitric oxide synthetase.

As used herein, the term "growth factor" refers to a peptide, protein, glycoprotein, lipoprotein, or a fragment or modification thereof, or a synthetic molecule, which stimulates endothelial, stem or progenitor cells to grow and differentiate into mature, functional endothelial cells. Mature endothelial cells express nitric oxide synthetase, thereby releasing nitric oxide into the tissues. Table 1 below lists some of the growth factors that can be used for coating the medical device.

**Table 1**

| Growth Factor | Endothelial cell specific |
|---|---|
| Acidic fibroblast growth factor (aFGF) | No |
| Basic fibroblast growth factor (bFGF) | No |
| Fibroblast growth factor 3 (FGF-3) | No |
| Fibroblast growth factor 4 (FGF-4) | No |
| Fibroblast growth factor 5 (FGF-5) | No |
| Fibroblast growth factor 6 (FGF-6) | No |
| Fibroblast growth factor 7 (FGF-7) | No |
| Fibroblast growth factor 8 (FGF-8) | No |
| Fibroblast growth factor 9 (FGF-9) | No |
| Angiogenin 1 | Yes |
| Angiogenin 2 | Yes |
| Hepatocyte growth factor / scatter factor (HGF/SF) | No |
| Platelet-derived growth factor (PDE-CGF) | Yes |
| Transforming growth factor-α (TGF-α) | No |
| Transforming growth factor-β (TGF-β) | No |
| Tumor necrosis factor-α (TNF-α) | No |
| Vascular endothelial growth factor 121 (VEGF 121) | Yes |
| Vascular endothelial growth factor 145 (VEGF 145) | Yes |
| Vascular endothelial growth factor 165 (VEGF 165) | Yes |
| Vascular endothelial growth factor 189 (VEGF 189) | Yes |
| Vascular endothelial growth factor 206 (VEGF 206) | Yes |
| Vascular endothelial growth factor B (VEGF-B) | Yes |
| Vascular endothelial growth factor C (VEGF-C) | Yes |
| Vascular endothelial growth factor D (VEGF-D) | Yes |
| Vascular endothelial growth factor E (VEGF-E) | Yes |
| Vascular endothelial growth factor F (VEGF-F) | Yes |
| Placental growth factor | Yes |
| Angiopoietin-1 | No |
| Angiopoietin-2 | No |
| Thrombospondin (TSP) | No |
| Proliferin | Yes |
| Ephrin-A1 (B61) | Yes |
| E-selectin | Yes |
| Chicken chemotactic and angiogenic factor (cCAF) | No |
| Leptin | Yes |
| Heparin affinity regulatory peptide (HARP) | No |
| Heparin | No |
| Granulocyte colony stimulating factor | No |
| Insulin-like growth factor | No |
| Interleukin 8 | No |
| Thyroxine | No |
| Sphingosine 1-phosphate | No |

As used herein, the term "VEGF" means any of the isoforms of the vascular endothelium growth factor listed in Table 1 above unless the isoform is specifically identified with its numerical or alphabetical abbreviation.

As used herein, the term "therapeutically effective amounts of growth factor" means the amount of a growth factor that stimulates or induces endothelial, progenitor or stem cells to grow and differentiate, thereby forming a confluent layer of mature and functional endothelial cells on the luminal surface of the medical device. The amount of a growth factor needed to practice the invention varies with the nature of the growth factor used and binding kinetics between the growth factor and its receptor. For example, 100 µg of VEGF has been shown to stimulate the adherence of endothelial cells on a medical device and form a confluent layer of epithelium. It is well known to those of ordinary skill in the art how to determine therapeutically effective amounts of a growth factor to use to stimulate cell growth and differentiation of endothelial cells.

As used herein, "intimal hyperplasia" is the undesirable increased in smooth muscle cell proliferation and matrix deposition in the vessel wall. As used herein "restenosis" refers to the reoccurrent narrowing of the blood vessel lumen. Vessels may become obstructed because of restenosis. After PTCA or PTA, smooth muscle cells from the media and adventitia, which are not normally present in the intima, proliferate and migrate to the intima and secrete proteins, forming an accumulation of smooth muscle cells and matrix protein within the intima. This accumulation causes a narrowing of the lumen of the artery, reducing blood flow distal to the narrowing. As used herein, "inhibition of restenosis" refers to the inhibition of migration and proliferation of smooth muscle cells accompanied by prevention of protein secretion so as to prevent restenosis and the complications arising therefrom.

The subjects that can be treated using the medical device, methods and compositions of this invention are mammals, or more specifically, a human, dog, cat, pig, rodent or monkey.

The methods of the present invention may be practiced *in vivo* or *in vitro.*

The term "progenitor endothelial cell" refers to endothelial cells at any developmental stage, from progenitor or stem cells to mature, functional epithelial cells from bone marrow, blood or local tissue origin and which are non-malignant.

For *in vitro* studies or use of the coated medical device, fully differentiated endothelial cells may be isolated from an artery or vein such as a human umbilical vein, while progenitor endothelial cells are isolated from peripheral blood or bone marrow. The endothelial cells are bound to the medical devices by incubation of the endothelial cells with a medical device coated with the matrix that incorporates an antibody, a growth factor, or other agent that adheres to endothelial cells. In another embodiment, the endothelial cells can be transformed endothelial cells. The transfected endothelial cells contain vectors which express growth factors or proteins which inhibit thrombogenesis, restenosis, or any other therapeutic end.

### Endothelial Cells

Human umbilical vein endothelial cells (HUVEC) are obtained from umbilical cords according to the methods of Jaffe, et al., J. Clin. Invest., 52:2745-2757, 1973, and were used in the experiments. Briefly, cells are stripped from the blood vessel walls by treatment with collagenase and cultured in gelatin-coated tissue culture flasks in M199 medium containing 10% low endotoxin fetal calf serum, 90 ug/ml preservative-free porcine heparin, 20 ug/ml endothelial cell growth supplement (ECGS) and glutamine.

Progenitor endothelial cells (EPC) are isolated from human peripheral blood according to the methods of Asahara et al. (Isolation of putative progenitor endothelial cells for angiogenesis. Science 275:964-967, 1997). Magnetic beads coated with antibody to CD34 are incubated with fractionated human peripheral blood. After incubation, bound cells are eluted and can be cultured in EBM-2 culture medium. (Clonetics, San Diego, CA). Alternatively enriched medium isolation can be used to isolate these cells. Briefly, peripheral venous blood is taken from healthy male volunteers and the mononuclear cell fraction is isolated by density gradient centrifugation, and the cells are plated on fibronectin coated culture slides (Becton Dickinson) in EC basal medium-2 (EBM-2) (Clonetics) supplemented with 5% fetal bovine serum, human VEGF-A, human fibroblast growth factor-2, human epidermal growth factor, insulin-like growth factor-1, and ascorbic acid. EPCs are grown for 7-days, with culture media changes every 48 hours. Cells are characterized by fluorescent antibodies to CD45, CD34, CD31, VEGFR-2, Tie-2, and E-selectin.

Mammalian cells are transfected with any expression vectors that contains any cloned genes encoding proteins such as platelet derived growth factor (PDGF), fibroblast growth factor (FGF), or nitric oxide synthase (NOS) using conventional methods. (See, for example, mammalian expression vectors and transfection kits commercially available from Stratagene, San Diego, CA). For example, purified porcine progenitor endothelial cells are transfected with vascular endothelial growth factor (VEGF) using an adenoviral expression vector expressing the VEGF cDNA according to the methods of Rosengart et al. (Six-month assessment of a phase I trial of angiogenic gene therapy for the treatment of coronary artery disease using direct intramyocardial administration of an adenovirus vector expressing the VEGF121 cDNA. Ann. Surg. 230(4):466-470 (1999)).

### Antibodies

Monoclonal antibodies useful in the method of the invention may be produced according to the standard techniques of Kohler and Milstein (Continuous cultures of fused cells secreting antibody of predefined specificity. Nature 265:495-497, 1975), or can be obtained from commercial sources. Endothelial cells can be used as the immunogen to produce monoclonal antibodies directed against endothelial cell surface antigens.

Monoclonal antibodies directed against endothelial cells are prepared by injecting HUVEC or purified progenitor endothelial cells into a mouse or rat. After a sufficient time, the mouse is sacrificed and spleen cells are obtained. The spleen cells are immortalized by fusing them with myeloma cells or with lymphoma cells, generally in the presence of a non-ionic detergent, for example, polyethylene glycol. The resulting cells, which include the fused hybridomas, are allowed to grow in a selective medium, such as HAT-medium, and the surviving cells are grown in such medium using limiting dilution conditions. The cells are grown in a suitable container, e.g., microtiter wells, and the supernatant is screened for monoclonal antibodies having the desired specificity, i.e., reactivity with endothelial cell antigens.

Various techniques exist for enhancing yields of monoclonal antibodies such as injection of the hybridoma cells into the peritoneal cavity of a mammalian host which accepts the cells and then harvesting the ascites fluid. Where an insufficient amount of monoclonal antibody collects in the ascites fluid, the antibody is harvested from the blood of the host. Various conventional ways exist for isolation and purification of monoclonal antibodies so as to free the monoclonal antibodies from other proteins and other contaminants.

Also included within the scope of the invention are useful binding fragments of anti-endothelial cell monoclonal antibodies such as the Fab, F(ab')₂ of these monoclonal antibodies. The antibody fragments are obtained by conventional techniques. For example, useful binding fragments may be prepared by peptidase digestion of the antibody using papain or pepsin.

Antibodies of the invention are directed to an antibody of the IgG class from a murine source; however, this is not meant to be a limitation. The above antibody and those antibodies having functional equivalency with the above antibody, whether from a murine source, mammalian source including human, or other sources, or combinations thereof are included within the scope of this invention, as well as other classes such as IgM, IgA, IgE, and the like, including isotypes within such classes. In the case of antibodies, the term "functional equivalency" means that two different antibodies each bind to the same antigenic site on an antigen, in other words, the antibodies compete for binding to the same antigen. The antigen may be on the same or different molecule.

In one embodiment, monoclonal antibodies reacting with the endothelial cell surface antigen CD34 are used. Anti-CD34 monoclonal antibodies attached to a solid support have been shown to capture progenitor endothelial cells from human peripheral blood. After capture, these progenitor cells are capable of differentiating into endothelial cells. (Asahara et al. 1997. Isolation of putative progenitor endothelial cells for angiogenesis. Science 275:964-967.) Hybridomas producing monoclonal antibodies directed against CD34 can be obtained from the American Type Tissue Collection. (Rockville, MD). In another embodiment, monoclonal antibodies reactive with endothelial cell surface antigens such as VEGFR-1 and VEGFR-2, CD133, or Tie-2 are used.

Polyclonal antibodies reactive against endothelial cells isolated from the same species as the one receiving the medical device implant may also be used.

### Stent

The term "stent" herein means any medical device which when inserted or implanted into the lumen of a vessel expands the cross-sectional lumen of a vessel. The term "stent" includes, stainless steel stents commercially available which have been coated by the methods of the invention; covered stents such as those covered with PTFE or ePTFE. In one embodiment, this includes stents delivered percutaneously to treat coronary artery occlusions or to seal dissections or aneurysms of the splenic, carotid, iliac and popliteal vessels. In another embodiment, the stent is delivered into a venous vessel. The stent can be composed of polymeric or metallic structural elements onto which the matrix comprising the antibodies and the compound, such as growth factors, is applied or the stent can be a composite of the matrix intermixed with a polymer. For example, a deformable metal wire stent can be used, such as that disclosed in U.S. Pat. No. 4,886,062 to Wiktor. A self-expanding stent of resilient polymeric material such as that disclosed in published international patent application WO91/12779 "Intraluminal Drug Eluting Prosthesis", can also be used. Stents may also be manufactured using stainless steel, polymers, nickel-titanium, tantalum, gold, platinum-iridium, or Elgiloy and MP35N and other ferrous materials. Stents are delivered through the body lumen on a catheter to the treatment site where the stent is released from the catheter, allowing the stent to expand into direct contact with the lumenal wall of the vessel. In another embodiment, the stent comprises a biodegradable stent (H. Tamai, pp 297 in Handbook_of_Coronary Stents,_3rd_Edition, Eds. PW Serruys and MJB Kutryk, Martin Dunitz (2000). It will be apparent to those skilled in the art that other self-expanding stent designs (such as resilient metal stent designs) could be used with the antibodies, growth factors and matrices of this invention.

### Synthetic Graft

The term "synthetic graft" means any artificial prosthesis having biocompatible characteristics. In one embodiment, the synthetic grafts can be made of polyethylene terephthalate (Dacron®, PET) or polytetrafluoroehtylene (Teflon®, ePTFE). In another embodiment, synthetic grafts are composed of polyurethane, cross-linked PVA hydrogel, and/or biocompatible foams of hydrogels. In yet a third embodiment, a synthetic graft is composed of an inner layer of meshed polycarbonate urethane and an outer layer of meshed polyethylene terephthalate. It will be apparent to those skilled in the art that any biocompatible synthetic graft can be used with the antibodies, growth factors, and matrices of this invention. (Bos et al. 1998. Small-Diameter Vascular Prostheses: Current Status. Archives Physio Biochem. 106:100-115). Synthetic grafts can be used for end-to-end, end to side, side to end, side to side or intraluminal and in anastomosis of vessels or for bypass of a diseased vessel segments, for example, as abdominal aortic aneurysm devices.

### Matrix

(A) Synthetic Materials - The matrix that is used to coat the stent or synthetic graft may be selected from synthetic materials such as polyurethane, segmented polyurethane-urea/heparin, poly-L-lactic acid, cellulose ester, polyethylene glycol, cross-linked PVA hydrogel, biocompatible foams of hydrogels, or hydrophilic dextrans, such as carboxymethyl dextran.
(B) Naturally Occurring Material - The matrix may be selected from naturally occurring substances such as collagen, fibronectin, vitronectin, elastin, laminin, heparin, fibrin, cellulose or carbon. A primary requirement for the matrix is that it be sufficiently elastic and flexible to remain unruptured on the exposed surfaces of the stent or synthetic graft.
(C) Fullerenes - The matrix may also comprise a fullerene (the term "fullerene" encompasses a plurality of fullerene molecules). Fullerenes are carbon-cage molecules. The number of carbon (C) molecules in a fullerene species varies from about C₂₀ to about C₁₅₀. Fullerenes are produced by high temperature reactions of elemental carbon or of carbon-containing species by processes well known to those skilled in the art; for example, by laser vaporization of carbon, heating carbon in an electric arc or burning of hydrocarbons in sooting flames. (U.S. Patent No. 5,292,813, to Patel et al.; U.S. Patent No. 5,558,903 to Bhushan et al.). In each case, a carbonaceous deposit or soot is produced. From this soot, various fullerenes are obtained by extraction with appropriate solvents, such as toluene. The fullerenes are separated by known methods, in particular by high performance liquid chromatography (HPLC). Fullerenes may be synthesized or obtained commercially from Dynamic Enterprises, Ltd., Berkshire, England or Southern Chemical Group, LLC, Tucker, Georgia, or Bucky USA, Houston Texas.

Fullerenes may be deposited on surfaces in a variety of different ways, including, sublimation, laser vaporization, sputtering, ion beam, spray coating, dip coating, roll-on or brush coating as disclosed in U.S. Patent No. 5,558,903, or by derivatization of the surface of the stent.

An important feature of fullerenes is their ability to form "activated carbon." The fullerene electronic structure is a system of overlapping pi-orbitals, such that a multitude of bonding electrons are cooperatively presented around the surface of the molecule. (Chemical and Engineering News, Apr. 8, 1991, page 59). As forms of activated carbon, fullerenes exhibit substantial van der Waals forces for weak interactions. The adsorptive nature of the fullerene surface may lend itself to additional modifications for the purpose of directing specific cell membrane interactions. For example, specific molecules that possess chemical properties that selectively bind to cell membranes of particular cell types or to particular components of cell membranes, e.g., lectins or antibodies, can be adsorbed to the fullerene surface. Attachment of different molecules to the fullerene surface may be manipulated to create surfaces that selectively bind various cell types, e.g., progenitor endothelial cells, epithelial cells, fibroblasts, primary explants, or T-cell subpopulations. U.S. Patent No. 5,310,669 to Richmond et al.; Stephen R. Wilson, Biological Aspects of Fullerenes, Fullerenes:Chemistry, Physics and Technology, Kadish et al. eds., John Wiley & Sons, NY 2000.

Fullerenes may also form nanotubes that incorporate other atoms or molecules. (Liu et al. Science 280:1253-1256 (1998)). The synthesis and preparation of carbon nanotubes is well known in the art. (U.S. Patent No. 5,753,088 to Olk et al., and U.S. Patent No. 5,641,466 to Ebbsen et al.) Molecules such as proteins can also be incorporated inside carbon nanotubes. For example, nanotubes may be filled with the enzymes, e.g., Zn₂Cd₂-metallothionein, cytochromes C and C3, and beta-lactamase after cutting the ends of the nanotube. (Davis et al. Inorganica Chim. Acta 272:261 (1998); Cook et al. Full Sci. Tech. 5(4):695 (1997)).

Three dimensional fullerene structures can also be used. U.S. Patent No. 5,338,571 to Mirkin et al., discloses three-dimensional, multilayer fullerene structures that are formed on a substrate surface by (i) chemically modifying fullerenes to provide a bond-forming species; (ii) chemically treating a surface of the substrate to provide a bond-forming species effective to covalently bond with the bond-forming species of the fullerenes in solution; and, (iii) contacting a solution of modified fullerenes with the treated substrate surface to form a fullerene layer covalently bonded to the treated substrate surface.

### (D) Application of the Matrix to the Medical Device

The matrix should adhere tightly to the surface of the stent or synthetic graft. Preferably, this is accomplished by applying the matrix in successive thin layers. Alternatively, antibodies and growth factors are applied only to the surface of the outer layer in direct contact with the vessel lumen. Different types of matrices may be applied successively in succeeding layers. The antibodies may be covalently or noncovalently coated on the matrix after application of the matrix to the stent.

In order to coat a medical device such as a stent, the stent is dipped or sprayed with a liquid solution of the matrix of moderate viscosity. After each layer is applied, the stent is dried before application of the next layer. In one embodiment, a thin, paint-like matrix coating does not exceed an overall thickness of 100 microns.

In one embodiment, the stent surface is first functionalized, followed by the addition of a matrix layer. Thereafter, the antibodies and the growth factor are coupled to the surface of the matrix. In this aspect of the invention, the techniques of the stent surface creates chemical groups which are functional. The chemical groups such as amines, are then used to immobilize an intermediate layer of matrix, which serves as support for the antibodies and the growth factor.

In another embodiment, a suitable matrix coating solution is prepared by dissolving 480 milligrams (mg) of a drug carrier, such as poly-D, L-lactid (available as R203 of Boehringer Inc., Ingelheim, Germany) in 3 milliliters (ml) of chloroform under aseptic conditions. In principle, however, any biodegradable (or non-biodegradable) matrix that is blood-and tissue-compatible (biocompatible) and can be dissolved, dispersed or emulsified may be used as the matrix if, after application, it undergoes relatively rapid drying to a self-adhesive lacquer- or paint-like coating on the medical device.

For example, coating a stent with fibrin is well known to one of ordinary skill in the art. In U.S. Patent No. 4,548,736 issued to Muller et al., fibrin is clotted by contacting fibrinogen with thrombin. Preferably, the fibrin in the fibrin-containing stent of the present invention has Factor XIII and calcium present during clotting, as described in U.S. Pat. No. 3,523,807 issued to Gerendas, or as described in published European Patent Application 0366564, in order to improve the mechanical properties and biostability of the implanted device. Preferably, the fibrinogen and thrombin used to make fibrin in the present invention are from the same animal or human species as that in which the stent will be implanted in order to avoid any inter-species immune reactions, e.g., human anti-cow. The fibrin product can be in the form of a fine, fibrin film produced by casting the combined fibrinogen and thrombin in a film and then removing moisture from the film osmotically through a semipermeable membrane. In the European Patent Application 0366564, a substrate (preferably having high porosity or high affinity for either thrombin or fibrinogen) is contacted with a fibrinogen solution and with a thrombin solution. The result is a fibrin layer formed by polymerization of fibrinogen on the surface of the medical device. Multiple layers of fibrin applied by this method could provide a fibrin layer of any desired thickness. Alternatively, the fibrin can first be clotted and then ground into a powder which is mixed with water and stamped into a desired shape in a heated mold (U.S. Patent No. 3,523,807). Increased stability can also be achieved in the shaped fibrin by contacting the fibrin with a fixing agent such as glutaraldehyde or formaldehyde. These and other methods known by those skilled in the art for making and forming fibrin may be used in the present invention.

If a synthetic graft is coated with collagen, the methods for preparing collagen and forming it on synthetic graft devices are well known as set forth in U.S. Patent No. 5,851,230 to Weadock et al. This patent describes methods for coating a synthetic graft with collagen. Methods for adhering collagen to a porous graft substrate typically include applying a collagen dispersion to the substrate, allowing it to dry and repeating the process. Collagen dispersions are typically made by blending insoluble collagen (approximately 1-2% by weight) in a dispersion at acidic pH (a pH in a range of 2 to 4). The dispersion is typically injected via syringe into the lumen of a graft and massaged manually to cover the entire inner surface area with the collagen slurry. Excess collagen slurry is removed through one of the open ends of the graft. Coating and drying steps are repeated several times to provide sufficient treatment.

In yet another embodiment, the stent or synthetic graft is coated with amorphous carbon. In U.S. Patent No. 5,198,263, a method for producing a high-rate, low-temperature deposition of amorphous carbon films in the presence of a fluorinated or other halide gas is described. Deposition according to the methods of this invention can be performed at less than 100° C, including ambient room temperature, with a radio-frequency, plasma-assisted, chemical-vapor deposition process. The amorphous carbon film produced using the methods of this invention adheres well to many types of substrates, including for example glasses, metals, semiconductors, and plastics.

Attachment of a fullerene moiety to reactive amino group sites of an amine-containing polymer to form the fullerene-graft, amine-containing polymers may be performed as described in U.S. Patent No. 5,292,813. Chemical modification in this manner allows for direct incorporation of the fullerenes into the stent. In another embodiment, the fullerenes may be deposited on the surface of the stent or synthetic grafts as described above. (see, WO 99/32184 to Leone et al. Fullerenes (e.g., C₆₀) may also be attached through an epoxide bond to the surface of stainless steel (Yamago et al., Chemical Derivatization of Organofullerenes through Oxidation, Reduction and C-O and C-C Bond Forming Reactions. J. Org. Chem., 58 4796-4798 (1998)). The attachment is through a covalent linkage to the oxygen. This compound and the protocols for coupling are commercially available from BuckyUSA. (BuckyUSA, Houston, Texas).

(E) Addition of Antibodies and growth factor to the Matrix - Antibodies that promote adherence of progenitor endothelial cells, and growth factors for promoting cell growth and differentiation are incorporated into the matrix, either covalently or noncovalently. Antibodies and growth factor may be incorporated into the matrix layer by mixing the antibodies and growth factor with the matrix coating solution and then applied to the surface of the device. Usually, antibodies and growth factors are attached to the surface of the outermost layer of matrix that is applied on the luminal surface of the device, so that the antibodies and growth factor are projecting on the surface that is in contact with the circulating blood. Antibodies and growth factors are applied to the surface matrix using standard techniques.

In one embodiment, the antibodies are added to a solution containing the matrix. For example, Fab fragments on anti-CD34 monoclonal antibody are incubated with a solution containing human fibrinogen at a concentration of between 500 and 800 mg/dl. It will be appreciated that the concentration of anti-CD34 Fab fragment will vary and that one of ordinary skill in the art could determine the optimal concentration without undue experimentation. The stent is added to the Fab/fibrin mixture and the fibrin activated by addition of concentrated thrombin (at a concentration of at least 1000U/ml). The resulting polymerized fibrin mixture containing the Fab fragments incorporated directly into the matrix is pressed into a thin film (less than 100 µm) on the surface of the stent or synthetic graft. Virtually any type of antibody or antibody fragment can be incorporated in this manner into a matrix solution prior to coating of a stent or synthetic graft.

For example, in another embodiment, whole antibodies with or without antibody fragments and growth factors are covalently coupled to the matrix. In one embodiment, the antibodies and growth factor(s) are tethered covalently the matrix through the use of hetero- or homobifunctional linker molecules. As used herein the term "tethered" refers to a covalent coupling of the antibody to the matrix by a linker molecule. The use of linker molecules in connection with the present invention typically involves covalently coupling the linker molecules to the matrix after it is adhered to the stent. After covalent coupling to the matrix, the linker molecules provide the matrix with a number of functionally active groups that can be used to covalently couple one or more types of antibody. FIG. 1A provides an illustration of coupling via a cross-linking molecule. An endothelial cell, 1.01, binds to an antibody, 1.03, by a cell surface antigen, 1.02. The antibody is tethered to the matrix, 1.05-1.06, by a cross-linking molecule, 1.04. The matrix, 1.05-1.06, adheres to the stent, 1.07. The linker molecules may be coupled to the matrix directly (i.e., through the carboxyl groups), or through well-known coupling chemistries, such as, esterification, amidation, and acylation. The linker molecule may be a di- or tri-amine functional compound that is coupled to the matrix through the direct formation of amide bonds, and provides amine-functional groups that are available for reaction with the antibodies. For example, the linker molecule could be a polyamine functional polymer such as polyethyleneimine (PEI), polyallylamine (PALLA) or polyethyleneglycol (PEG). A variety of PEG derivatives, e.g., mPEG-succinimidyl propionate or mPEG-N-hydroxysuccinimide, together with protocols for covalent coupling, are commercially available from Shearwater Corporation, Birmingham, Alabama. (See also, Weiner et al., Influence of a poly-ethyleneglycol spacer on antigen capture by immobilized antibodies. J. Biochem. Biophys. Methods 45:211-219 (2000)). It will be appreciated that the selection of the particular coupling agent may depend on the type of antibody used and that such selection may be made without undue experimentation. Mixtures of these polymers can also be used. These molecules contain a plurality of pendant amine-functional groups that can be used to surface-immobilize one or more antibodies.

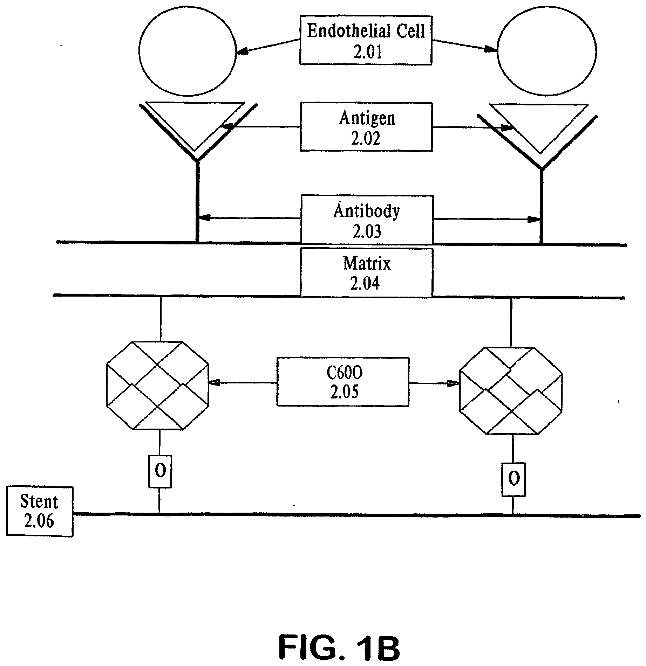

Antibodies may be attached to C₆₀ fullerene layers that have been deposited directly on the surface of the stent. Cross linking agents may be covalently attached to the fullerenes. The antibodies are then attached to the cross-linking agent, which in turn is attached to the stent. FIG. 1B provides an illustration of coupling by C₆₀. The endothelial cell, 2.01, is bound via a cell surface antigen, 2.02, to an antibody, 2.03, which in turn is bound, covalently or non-covalently to the matrix, 2.04. The matrix, 2.04, is coupled covalently via C₆₀, 2.05, to the stent, 2.06.

Small molecules of the invention comprise synthetic or naturally occurring molecules or peptides which can be used in place of antibodies, growth factors or fragments thereof. For example, lectin is a sugar-binding peptide of non-immune origin which occurs naturally. The endothelial cell specific Lectin antigen (Ulex Europaeus Uea 1) (Schatz et al. 2000 Human Endometrial Endothelial Cells: Isolation, Characterization, and Inflammatory-Mediated Expression of Tissue Factor and Type 1 Plasminogen Activator Inhibitor. Biol Reprod 62: 691-697) can selectively bind the cell surface of progenitor endothelial cells.

Synthetic "small molecules" have been created to target various cell surface receptors. These molecules selectively bind a specific receptor(s) and can target specific cell types such as progenitor endothelial cells. Small molecules can be synthesized to recognize endothelial cell surface markers such as VEGF. SU11248 (Sugen Inc.) (Mendel et al. 2003 In vivo antitumor activity of SU11248, a novel tyrosine kinase inhibitor targeting vascular endothelial growth factor and platelet-derived growth factor receptors: determination of a pharmacokinetic/pharmacodynamic relationship. Clin Cancer Res. Jan;9(1):327-37), PTK787/ZK222584 (Drevs J. et al. 2003 Receptor tyrosine kinases: the main targets for new anticancer therapy. Curr Drug Targets. Feb;4(2):113-21) and SU6668 (Laird, AD et al. 2002 SU6668 inhibits Flk-1/KDR and PDGFRbeta in vivo, resulting in rapid apoptosis of tumor vasculature and tumor regression in mice. FASEB J. May;16(7):681-90) are small molecules which bind to VEGFR-2.

Another subset of synthetic small molecules which target the endothelial cell surface are the alpha(v)beta(3) integrin inhibitors. SM256 and SD983 (Kerr JS. et al. 1999 Novel small molecule alpha v integrin antagonists: comparative anti-cancer efficacy with known angiogenesis inhibitors. Anticancer Res Mar-Apr;19(2A):959-68) are both synthetic molecules which target and bind to alpha(v)beta(3) present on the surface of endothelial cells.

### EXPERIMENTAL EXAMPLES

This invention is illustrated in the experimental details section which follows. These sections set forth below the understanding of the invention, but are not intended to, and should not be construed to, limit in any way the invention as set forth in the claims which follow thereafter.

### EXAMPLE 1

### Endothelial Progenitor Cell Phenotyping

Endothelial Progenitor Cells (EPC) were isolated either by CD34+ Magnetic Bead Isolation (Dynal Biotech) or enriched medium isolation as described recently (Asahara T, Murohara T, Sullivan A, et al. Isolation of putative progenitor endothelial cells for angiogenesis. Science 1997;275:964-7). Briefly, peripheral venous blood was taken from healthy male volunteers and the mononuclear cell fraction was isolated by density gradient centrifugation, and the cells were plated on human fibronectin coated culture slides (Becton Dickinson) in EC basal medium-2 (EBM-2) (Clonetics) supplemented with 5% fetal bovine serum, human VEGF-A, human fibroblast growth factor-2, human epidermal growth factor, insulin-like growth factor-1, and ascorbic acid. EPCs were grown up to seven days with culture media changes every 48 hours. The results of these experiments are shown in FIGs. 2A and 2B. FIGs. 2A and 2B show that the anti-CD34 isolated cell appear more spindle-like, which indicates that the cells are differentiating into endothelial cells.

EC phenotype was determined by immunohistochemistry. Briefly, EPC were fixed in 2% Paraformaldehyde (PFA) (Sigma) in Phosphate buffered saline (PBS) (Sigma) for 10 minutes, washed 3X with PBS and stained with various EC specific markers; rabbit anti-human VEGFR-2 (Alpha Diagnostics Intl. Inc.), mouse anti-human Tie-2 (Clone Ab33, Upstate Biotechnology), mouse anti-human CD34 (Becton Dickinson), EC-Lectin (Ulex Europaeus Uea 1) (Sigma) and mouse anti-human Factor 8 (Sigma). The presence of antibody was confirmed by exposure of the cells to a fluorescein isothiocyanate-conjugated (FITC) secondary antibody. Propidium Iodine (PI) was used as a nuclear marker. The results of these experiments are shown in FIGs. 2C-2G. FIG. 2C shows that VEGFR-2 is expressed after 24 hours in culture, confirming that the cells are endothelial cells. FIGs. 2D and 2F show the nuclear staining of the bound cells after 7 days of incubation and FIGs. 2E and 2G the same field of cells stained with and anti-Tie-2 antibody.

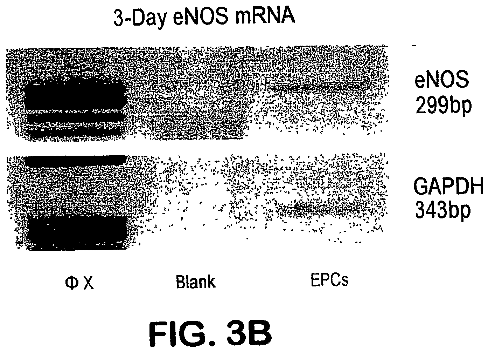

EPCs ability to express endothelial nitric oxide synthase (eNOS), a hallmark of EC function, was determined by Reverse Transcriptase-Polymerase Chain Reaction (rt-PCR) for eNOS mRNA. EPCs were grown up to seven days in EBM-2 medium after which total RNA was isolated using the GenElute Mammalian total RNA kit (Sigma) and quantified by absorbance at 260 nm. Total RNA was reverse transcribed in 20 µL volumes using Omniscript RT kit (Qiagen) with 1 µg of random primers. For each RT product, aliquots (2-10 µL) of the final reaction volume were amplified in two parallel PCR reactions using eNOS (299 bp product, sense 5'-TTCCGGGGATTCTGGCAGGAG-3', antisense 5'-GCCATGGTAACATCGCCGCAG-3') or GAPDH (343 bp product, sense 5'-CTCTAAGGCTGTGGGCAAGGTCAT-3', antisense 5'-GAGATCCACCACCCTGTTGCTGTA-3') specific primers and Taq polymerase (Pharmacia Biotech Amersham). PCR cycles were as follows: 94°C for 5 minutes, 65°C for 45 seconds, 72°C for 30 seconds (35 cycles for eNOS and 25 cycles for GAPDH). rt-PCR products were analyzed by 2% agarose gel electrophoresis, visualized using ethidium bromide and quantified by densitometry. The results of this experiment are shown in FIGs. 3A and 3B. As seen in FIGs. 3A and 3B, nitric oxide synthetase (eNOS) is express after the cells have been incubated in medium for 3 days in culture in the presence or absence of oxygen. eNOS mRNA expression continues to be present after 7-days in culture. The presence of eNOS mRNA indicates that the cells have differentiated into mature endothelial cells by day 3 and have begun to function like fully differentiated endothelial cells.

### EXAMPLE 2

**Endothelial Cell Capture by anti-CD34 coated Stainless Steel** Disks: Human Umbilical Vein Endothelial Cells (HUVEC) (American Type Culture Collection) are grown in endothelial cell growth medium for the duration of the experiments. Cells are incubated with CMDX and gelatin coated samples with or without bound antibody on their surface or bare stainless steel (SST) samples. After incubation, the growth medium is removed and the samples are washed twice in PBS. Cells are fixed in 2% paraformaldehyde (PFA) for 10 minutes and washed three times, 10 minutes each wash, in PBS, to ensure all the fixing agent is removed. Each sample is incubated with blocking solution for 30 minutes at room temperature, to block all non-specific binding. The samples are washed once with PBS and the exposed to 1:100 dilution of VEGFR-2 antibody and incubated overnight. The samples are subsequently washed three times with PBS to ensure all primary antibody has been removed. FITC-conjugated secondary antibody in blocking solution is added to each respective sample at a dilution of 1:100 and incubated for 45 minutes at room temperature on a Belly Dancer apparatus. After incubation, the samples are washed three times in PBS, once with PBS containing 0.1% Tween 20, and then again in PBS. The samples are mounted with Propidium Iodine (PI) and visualized under confocal microscopy.

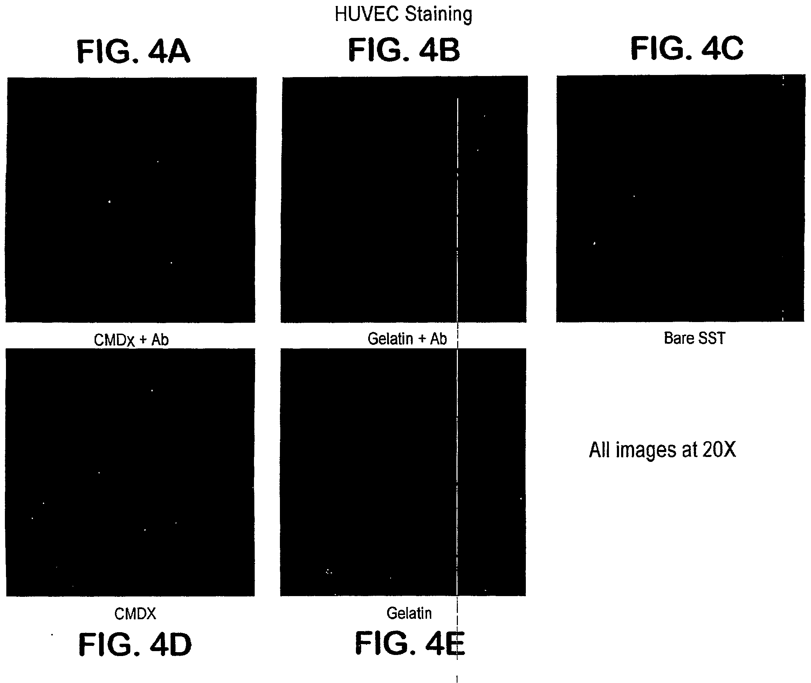

FIGs. 4A-4E are photomicrographs of SST samples coated with CMDX and anti-CD34 antibody (FIG. 4A), gelatin and anti-CD34 antibody coated (FIG. 4B), bare SST (FIG. 4C), CMDX coated and no antibody (FIG, 4D) and gelatin-coated and no antibody (FIG. 4E). The figures show that only the antibody coated samples contain numerous cells attached to the surface of the sample as shown by PI staining. The bare SST control disk shows few cells attached to its surface.

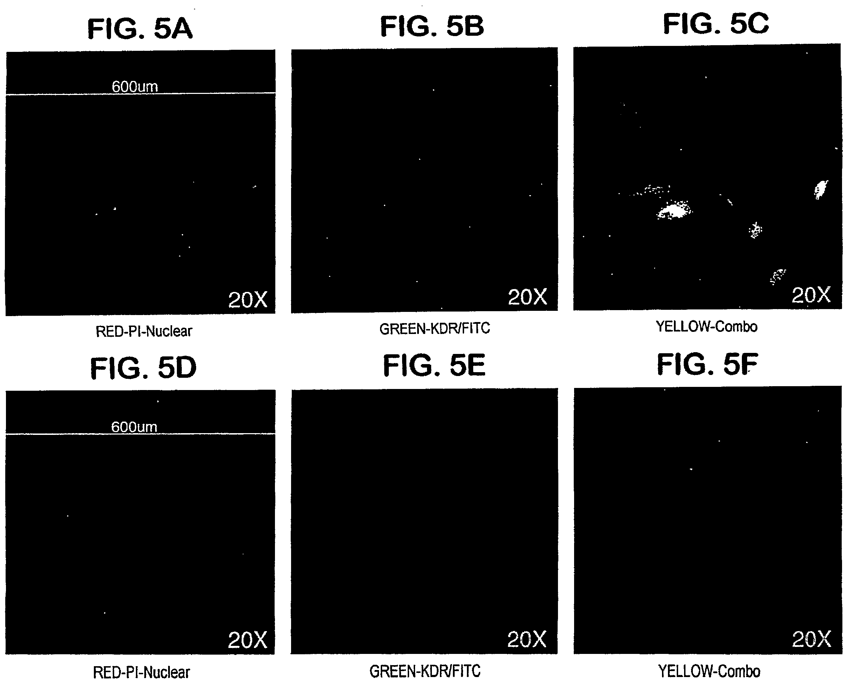

FIGs. 5A-5C are photomicrographs of control samples CMDX-coated without antibody bound to its surface. FIG. 5A shows very few cells as seen by PI staining adhered to the surface of the sample. FIG. 5B shows that the adherent cells are VEGFR-2 positive indicating that they are endothelial cells and FIG. 5C shows a combination of the stained nuclei and the VEGFR-2 positive green fluorescence. FIGs. 5D-F are photomicrographs of control samples coated with gelatin without antibody on its surface. FIG. 5D shows no cells are present since PI staining is not present in the sample and there is no green fluorescence emitted by the samples (see FIGs. 5E and 5F).

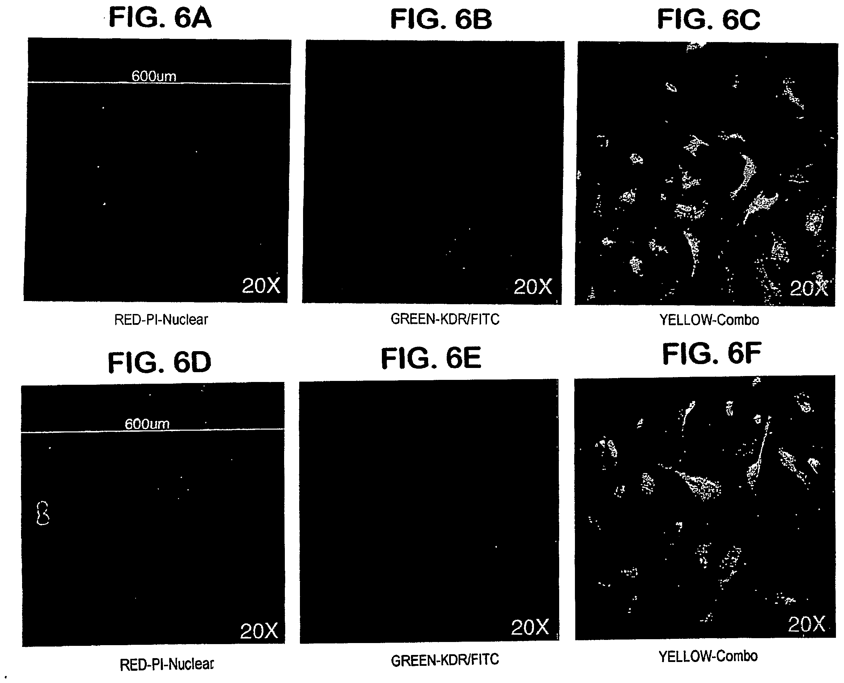

FIGs. 6A-6C are photomicrographs of CMDX coated SST samples having anti-CD34 antibody bound on its surface. The figures show that the samples contain numerous adherent cells which have established a near confluent monolayer (FIG. 6A) and which are VEGFR-2 positive (FIGs. 6B and 6C) as shown by the green fluorescence. Similarly, FIGs. 6D-6F are photomicrographs of a gelatin-coated sample with anti-CD34 antibody bound to its surface. These figures also show that HUVECs attached to the surface of the sample as shown by the numerous red-stained nuclei and green fluorescence from the VEGFR-2/FITC antibody (FIGs. 6E and 6F).

### EXAMPLE 3

**VEGFR-2 and Tie-2 Staining of Progenitor Endothelial Cells:**
Progenitor cell are isolated from human blood as described in the in Example 1 and incubated in growth medium for 24 hours, 7 days, and 3 weeks *in vitro.* After incubation, the growth medium is removed and the samples are washed twice in PBS. Cells are fixed in 2% paraformaldehyde (PFA) for 10 minutes and washed three times, 10 minutes each wash, in PBS, to ensure all the fixing agent is removed. Each sample is incubated with 440 µl of Goat (for VEGFR-2) or Horse (for Tie-2) blocking solution for 30 minutes at room temperature, to block all non-specific binding. The samples are washed once with PBS and the VEGFR-2 or Tie-2 antibody was added at a dilution of 1:100 in blocking solution and the samples are incubated overnight. The samples are then washed three times with PBS to ensure all primary antibody has been washed away. FITC-conjugated secondary antibody (200 µl) in horse or goat blocking solution is added to each respective sample at a dilution of 1:100 and incubated for 45 minutes at room temperature on a Belly Dancer apparatus. After incubation, the samples are washed three times in PBS, once with PBS containing 0.1 % Tween 20, and then again in PBS. The samples are mounted with Propidium Iodine (PI) and visualized under confocal microscopy.

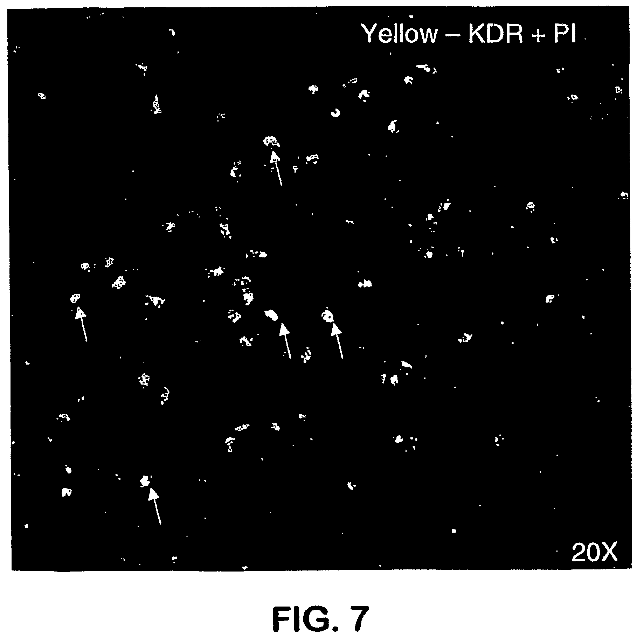

FIG.7 is a photomicrograph of a CMDX-coated sample containing CD34 antibody on its surface which was incubated with the cells for 24 hours, and shows that progenitor cells were captured on the surface of the sample and as demonstrated by the red-stained nuclei present on the surface of the sample. The figure also shows that about 75% of the cells are VEGFR-2 positive with a round morphology.

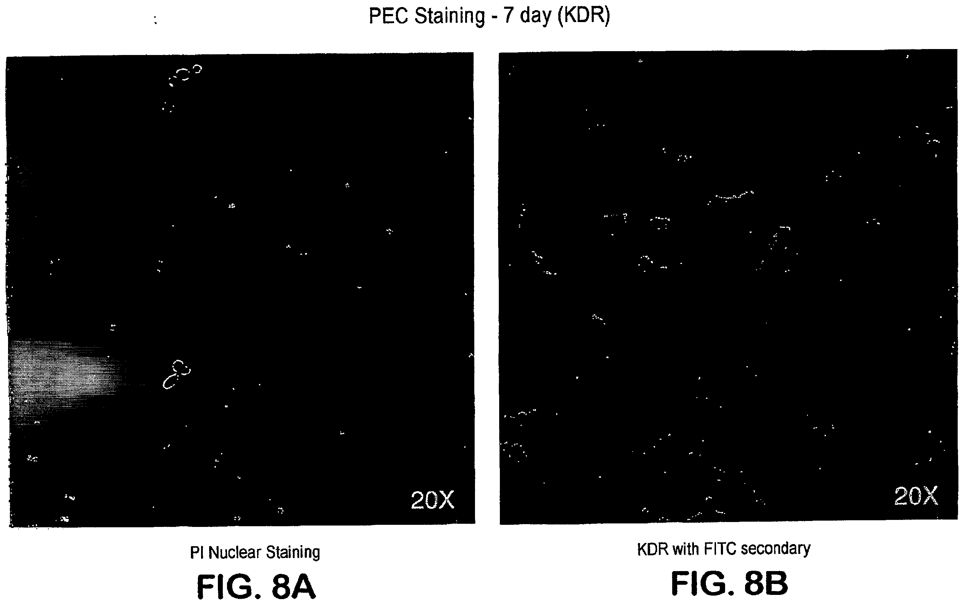

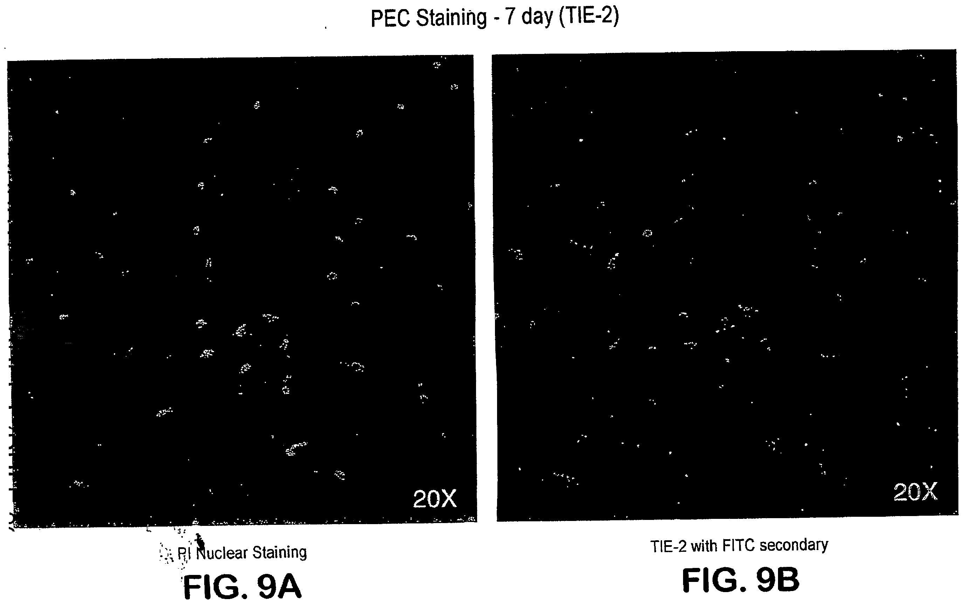

FIGs. 8A and 8B are from a sample which was incubated with the cells for 7 days. As seen in FIG. 8A, there are cells present on the sample as shown by the red-stained nuclei, which are VEGFR-2 positive (FIG. 8B, 100%) and are more endothelial in structure as shown by the spindle shape of the cells. FIGs. 9A and 9B are photomicrographs of CMDX-coated sample containing CD34 antibody on its surface, which was incubated for 7 days with the cells and after incubation, the sample was exposed to Tie-2 antibody. As seen in FIGs. 9A, there are numerous cells attached to the surface of the samples as shown by the red-stained nuclei. The cells adhered to the sample are also Tie-2 positive (100%) as seen by the green fluorescence emitted from the cells (FIG. 9B). In summary, after 7 days of incubation of the cells with the samples, the CD34 antibody-coated samples are able to capture endothelial cells on their surface as seen by the numerous cells attached to the surface of the samples and the presence of VEGFR-2 and Tie-2 receptors on the surface of the adhered cells. In addition, the presence of 100% endothelial cells on the surface of the samples at 7 days indicates that the non-endothelial cells may have detached or that all adherent cells have begun to express endothelial cell markers by day 7.

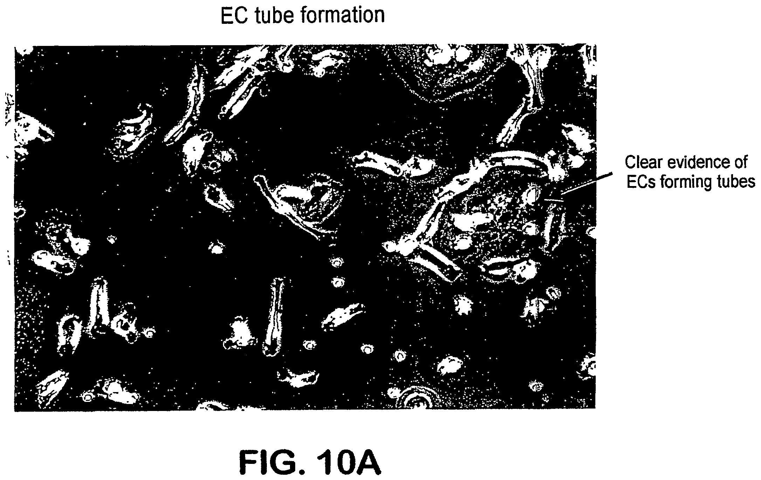

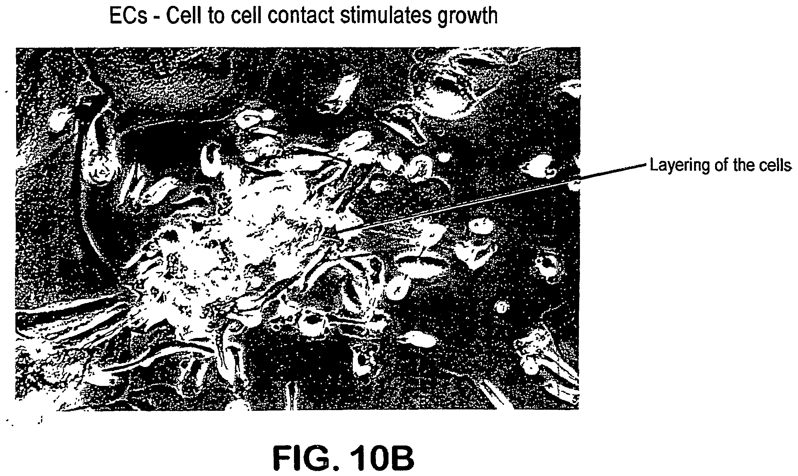

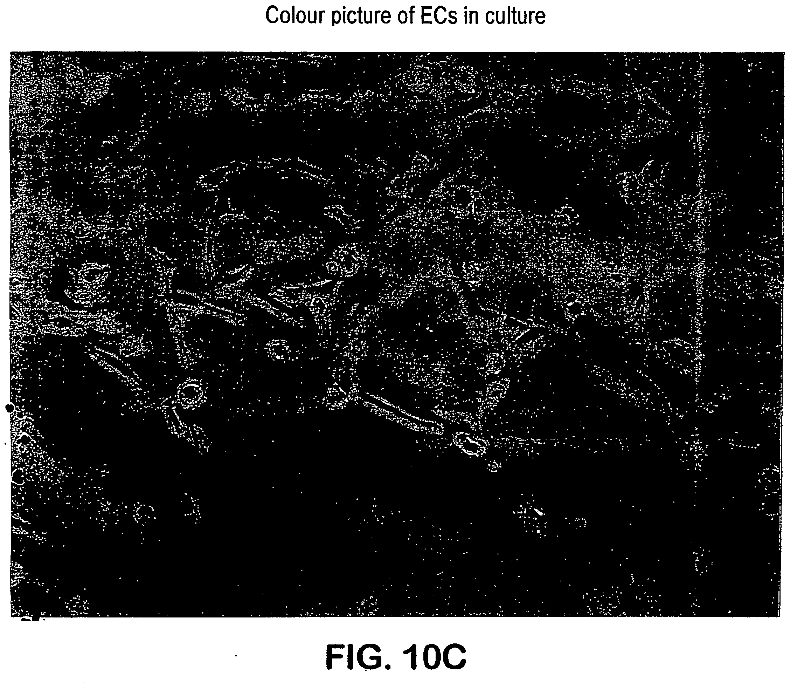

FIGs. 10A-10C are phase contrast photomicrographs of the progenitor endothelial cells grown for 3 weeks in endothelial cell growth medium. FIG. 10A demonstrates the cells have differentiated into matured endothelial cells as shown by the two-dimensional tube-like structures (arrow) reminiscent of a lumen of a blood vessel at the arrow. FIG. 10B shows that there is a three-dimensional build-up of cells in multiple layers; i.e.; one on top of the other, which confirms reports that endothelial cells grown for prolonged periods of time begin to form layers one on top of the other. FIG. 10C shows progenitor cells growing in culture 3 weeks after plating which have the appearance of endothelial cells, and the figure confirms that the cells are endothelial cells as demonstrated by the green fluorescence of the CD34/FITC antibodies present on their surface.

The above data demonstrate that white blood cells isolated from human blood have CD34 positive progenitor cells and that these cells can develop into mature endothelial cells and readily express endothelial cell surface antigens. (VEGFR-2 and Tie-2) The data also show that antibodies against progenitor or stem cell surface antigens can be used to capture these cells on the surface of a coated medical device of the invention.

### EXAMPLE 4

### Fullerene Coated and Fullerene Coated with anti-CD34 Antibody and/or an Endothelial Cell Growth Factor (Ang-2, VEGF) Stainless Steel

Stainless steel stents and disks are derivatized with a functional fullerene layer for attaching antibodies and/or growth factors (i.e., VEGF or Ang-2) using the following procedure:

In the first step, the surface of the SST stent or disk is activated with 0.5M HCL which also cleans the surface of any passivating contaminants. The metal samples are removed from the activation bath, rinsed with distilled water, dried with methanol and oven-dried at 75 °C. The stents are then immersed in the toluene derivative solution with fullerene oxide (C₆₀-O), for a period of up to 24 hours. The fullerene oxide binds to the stent via Fe-O, Cr-O and Ni-O found on the stent. The stents are removed from the derivatizing bath, rinsed with toluene, and placed in a Soxhlet Extractor for 16 hours with fresh toluene to remove any physisorbed C₆₀. The stents are removed and oven-dried at 105 °C overnight. This reaction yields a fully derivatized stent or disk with a monolayer of fullerenes.

In step 2 a di-aldehyde molecule is formed in solution by reacting sebacic acid with thionyl chloride or sulfur oxychloride (SOCl₂) to form Sebacoyl chloride. The resultant Sebacoyl chloride is reacted with LiAI[t-OButyl]₃ H and diglyme to yield 1,10-decanediol as shown below:

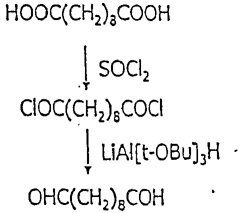

In step 3, an N-methyl pyrolidine derivate is formed on the surface of the stent or disk (from step 1). The fullerene molecule is further derivatized by reacting equimolar amounts of fullerene and N-methylglycine with the 1,10-decanediol product of the reaction of step 2, in refluxing toluene solution under nitrogen for 48 hours to yield N-methyl pyrolidine-derivatized fullerene-stainless steel stent or disk as depicted below.

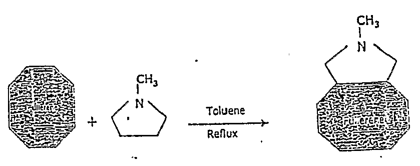

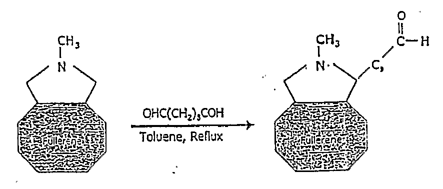

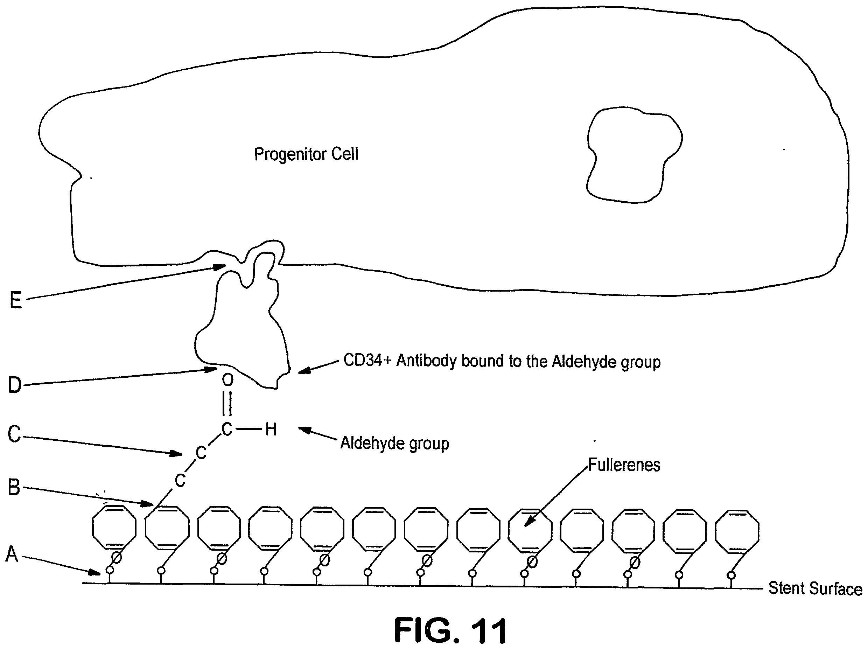

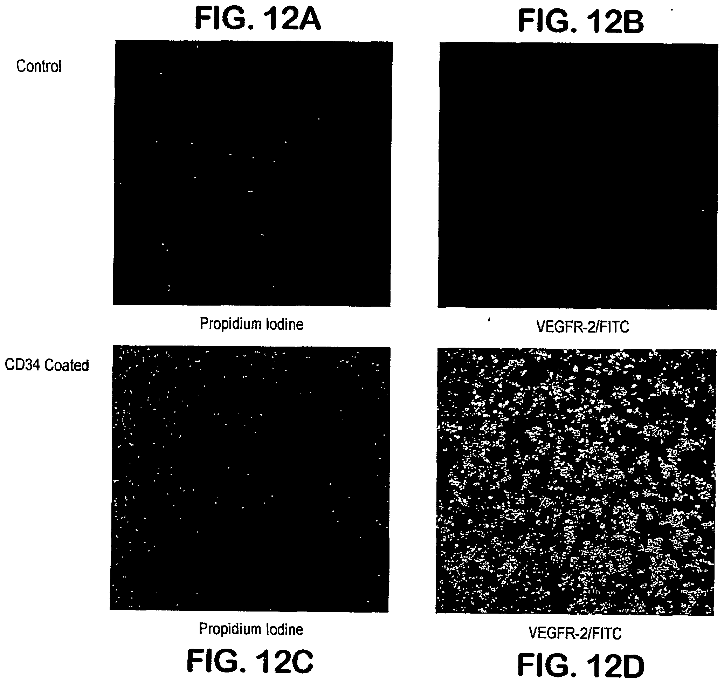

The derivatized stainless steel stent or disk is washed to remove any chemical residue and used to bind the antibodies and/or (VEGF or Ang-2) using standard procedures. Progenitor cell are isolated from human blood as described in Example 1 and exposed to the anti-CD34 antibody coated fullerene disks. After incubation, the growth medium is removed and the samples are washed twice in PBS. Cells are fixed in 2% paraformaldehyde (PFA) for 10 minutes and washed three times, 10 minutes each wash, in PBS, to ensure all the fixing agent is removed. Each sample is incubated with blocking solution for 30 minutes at room temperature, to block all non-specific binding. The samples are washed once with PBS and the exposed to 1:100 dilution of VEGFR-2 antibody and incubated overnight. The samples are subsequently washed three times with PBS to ensure all primary antibody has been removed. FITC-conjugated secondary antibody in blocking solution is added to each respective sample at a dilution of 1:100 and incubated for 45 minutes at room temperature on a Belly Dancer apparatus. After incubation, the samples are washed three times in PBS, once with PBS containing 0.1 % Tween 20, and then again in PBS. The samples are mounted with Propidium Iodine (PI) and visualized under confocal microscopy. FIG. 11 shows a schematic representation of a functional fullerene coated stent surface of the invention binding a progenitor cell. FIGs. 12A-12B are, respectively, photomicrographs of fullerene-coated control sample without antibody stained with PI (12A) and anti-VEGFR-2/FITC-conjugated antibody stained. FIGS. 12C and 12D are photomicrographs of a sample coated with a fullerene/anti-CD34 antibody coating. As shown in the figures, the anti-CD34 antibody coated sample contains more cells attached to the surface which are VEGFR-2 positive.

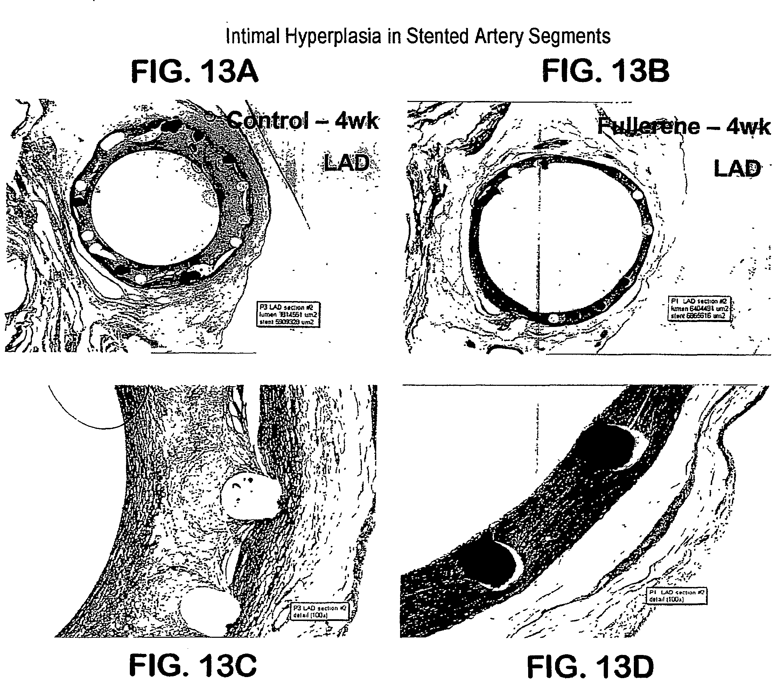

Fullerene-coated samples with and without antibodies are implanted into Yorkshire pigs as described in Example 5. The stents are explanted for histology and the stented segments are flushed with 10% buffered Formalin for 30 seconds followed by fixation with 10% buffered Formalin until processed. Five sections are cut from each stent; 1 mm proximal to the stent, 1 mm from the proximal end of the stent, mid stent, 1 mm from the distal edge of the stent and 1 mm distal to the stent. Sections are stained with Hematoxylin & Eosin (HE) and Elastin Trichrome. FIGs. 13A - 13D are photomicrographs of cross-sections through coronary artery explants of stents which had been implanted for 4 weeks. The data show that the fullerene-coated (FIGs. 13B and 13D) stents inhibit excessive intimal hyperplasia at the stent site over the control (bare stent, FIGs. 13A and 13C).

### EXAMPLE 5

**PORCINE BALLOON INJURY STUDIES:** Implantation of antibody-covered stents is performed in juvenile Yorkshire pigs weighing between 25 and 30 kg. Animal care complies with the "Guide for the Care and Use of Laboratory Animals" (NIH publication No. 80-23, revised 1985). After an overnight fast, animals are sedated with ketamine hydrochloride (20mg/kg). Following the induction of anesthesia with thiopental (12 mg/kg) the animals are intubated and connected to a ventilator that administers a mixture of oxygen and nitrous oxide (1:2 [vol/vol]). Anesthesia is maintained with 0.5-2.5 vol% isoflurane. Antibiotic prophylaxis is provided by an intramuscular injection of 1,000 mg of a mixture of procaine penicillin-G and benzathine penicillin-G (streptomycin).

Under sterile conditions, an arteriotomy of the left carotid artery is performed and a 8F-introducer sheath is placed in the left carotid artery. All animals are given 100 IU of heparin per kilogram of body weight. Additional 2,500 IU boluses of heparin are administered periodically throughout the procedure in order to maintain an activated clotting time above 300 seconds. A 6F guiding catheter is introduced through the carotid sheath and passed to the ostia of the coronary arteries. Angiography is performed after the administration of 200ug of intra coronary nitro glycerin and images analyzed using a quantitative coronary angiography system. A 3F-embolectomy catheter is inserted into the proximal portion of the coronary artery and passed distal to the segment selected for stent implantation and the endothelium is denuded. A coated R stent incorporating an anti-CD34 antibody is inserted through the guiding catheter and deployed in the denuded segment of the coronary artery. Bare stainless steel stents or stents coated with the matrix but without antibodies are used as controls. Stents are implanted into either the Left Anterior Descending (LAD) coronary artery or the Right Coronary Artery (RCA) or the Circumflex coronary artery (Cx) at a stent to artery ration of 1.1. The sizing and placement of the stents is evaluated angiographically and the introducer sheath was removed and the skin closed in two layers. Animals are placed on 300 mg of ASA for the duration of the experiment.

Animals are sacrificed at 1, 3, 7, 14, and 28 days after stent implantation. The animals are first sedated and anesthetized as described above. The stented coronary arteries are explanted with 1 cm of non-stented vessel proximal and distal to the stent. The stented arteries are processed in three ways, histology, immunohistochemistry or by Scanning Electron Microscopy.

For immunohistochemistry the dissected stents are gently flushed with 10% Formalin for 30seconds and the placed in a 10% Formalin/PBS solution until processing. Stents destined for immunohistochemistry are flushed with 2% Paraformaldehyde (PFA) in PBS for 30 seconds and then placed in a 2% PFA solution for 15min, washed and stored in PBS until immunohistochemistry with rabbit anti-human VEGFR-2 or mouse anti-human Tie-2 antibodies is performed.

Stents are prepared for SEM by flushing with 10% buffered Formalin for 30 seconds followed by fixation with 2% PFA with 2.5% glutaraldehyde in 0.1 M sodium cacodylate buffer overnight. Samples are then washed 3X with cacodylate buffer and left to wash overnight. Post-fixation was completed with 1% osmium tetroxide (Sigma) in 0.1 M cacodylate buffer which is followed by dehydration with ethanol (30% ethanol, 50%, 70%, 85%, 95%, 100%, 100%) and subsequent critical point drying with CO₂. After drying, samples are gold sputtered and visualized under SEM. (Reduction in thrombotic events with heparin-coated Palmaz-Schatz stents in normal porcine coronary arteries, Circulation 93:423-430).

For histology the stented segments are flushed with 10% buffered Formalin for 30seconds followed by fixation with 10% buffered Formalin until processed. Five sections are cut from each stent; 1 mm proximal to the stent, 1 mm from the proximal end of the stent, mid stent, 1 mm from the distal edge of the stent and 1 mm distal to the stent. Sections are stained with Hematoxylin & Eosin (HE) and Elastin Trichrome.

FIGs. 14A-14G show explants taken 1 (FIGs. 14A and 14B) and 48 hours (FIGs. 14C-14G) after implantation and observed under scanning electron microscope. The photomicrographs clearly show that the dextran/anti-CD34 antibody-coated stents (14B, 14E-G) have capture progenitor endothelial cells as shown by the spindle-shaped appearance of the cells at higher magnification (400X) at 48 hours compared to the dextran-coated control (14A, 14C and 14D).

Cross-sections of the explants from the swine coronary arteries also showed that the dextran-anti-CD34 antibody-coated (14L, 14M) caused a pronounced inhibition of intimal hyperplasia (thickness of the arterial smooth muscle layer) compared to the controls (bare stainless steel 14H and 14I; dextran-coated 14J and 14K). Fullerene-coated stent implants also inhibit intimal hyperplasia better than bare, control stainless steel stents as shown in FIGs. 13B-13D.

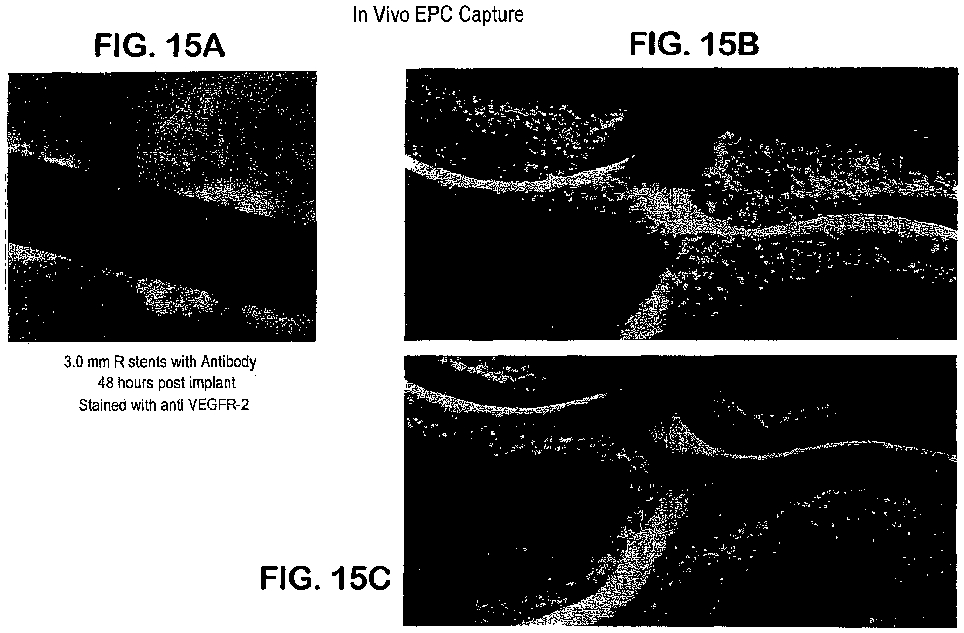

FIGs. 15A and 15B show, respectively, confocal photomicrographs of 48 hours explants of a dextran-plasma coated stent without antibody on is surface, and a dextran-plasma coated anti-CD34 antibody-stent of 18 mm in length. The stents had been implanted into the coronary artery of juvenile male Yorkshire swine. The explants were immunohistochemically processed and stained for VEGFR-2, followed by FITC-conjugated secondary antibody treatment and studied under confocal microscopy. FIGs. 15B and 15C show that the antibody containing stent is covered with endothelial cells as demonstrated by the green fluorescence of the section compared to the complete lack of endothelium on the stent without antibody (FIG. 15A).

### EXAMPLE 6

**Incorporation of an Endothelial Growth Factor into Immobilized Antibody Matrices Applied to Stents:** The following describes the steps for immobilizing an antibody directed toward endothelial progenitor cells cell surface antigens to a biocompatible matrix applied to an intravascular stent to which an endothelial growth factor is then absorbed for the enhanced attachment of circulating endothelial progenitor cells and their maturation to functional endothelium when in contact with blood.

Matrix Deposition: Using methods know to those skilled in the art, stainless steel stents are treated with a plasma deposition to introduce amine functionality on the stent surface. A layer of carboxy functional dextran (CMDX) will be bound to the amine functional layer deposited on the stent through the activation of the CMDX carboxyl groups using standard procedures, known as water soluble carbodiimide coupling chemistry, under aqueous conditions to which the amine groups on the plasma deposited layer to form an amide bond between the plasma layer and the functional CDMX.

Antibody Immobilization: Antibodies directed toward endothelial progenitor cells cell surface antigens, e.g., murine monoclonal anti-humanCD34, will be covalently coupled with the CDMX coated stents by incubation in aqueous water soluble carbodiimide chemistry in a buffered, acidic solution.

Absorption of Growth Factor: Subsequent to the immobilization of the monoclonal anti-humanCD34 to a CMDX matrix applied to a stent, the device is incubated in an aqueous solution of an endothelial growth factor, e.g. Angiopoietin-2, at an appropriate concentration such that the growth factor is absorbed into the CMDX matrix. The treated devices are rinsed in physiologic buffered saline solution and stored in a sodium azide preservative solution.

Using standard angiographic techniques, the above described devices when implanted in porcine coronary arteries and exposure to human blood produce an enhanced uptake and attachment of circulating endothelial progenitor cells on to the treated stent surface and accelerate their maturation into functional endothelium. The rapid establishment of functional endothelium is expected to decrease device thrombogenicity and modulate the extent of intimal hyperplasia.

### EXAMPLE 7

### Immobilization of an Endothelial Growth Factor and an

**Antibody on to Stents:** The following describes the steps for immobilizing an antibody directed toward endothelial progenitor cells cell surface antigens and an endothelial growth factor to a biocompatible matrix applied to an intravascular stent for the enhanced attachment of circulating endothelial progenitor cells and their maturation to functional endothelium when in contact with blood.

Matrix Deposition: Matrix Deposition: Using methods know to those skilled in the art, stainless steel stents are treated with a plasma deposition to introduce amine functionality on the stent surface. A layer of carboxy functional dextran (CMDX) is bound to the amine functional layer deposited on the stent through the activation of the CMDX carboxyl groups using standard procedures, known as water soluble carbodiimide coupling chemistry, under aqueous conditions to which the amine groups on the plasma deposited layer to form an amide bond between the plasma layer and the functional CDMX.

Antibody and Growth Factor Immobilization: Antibodies directed toward endothelial progenitor cells cell surface antigens, e.g. murine monoclonal anti-humanCD34, and an endothelial growth factor, e.g. Angiopoietin-2, is covalently coupled with the CDMX coated stents by incubation at equimolar concentrations in a water soluble carbodiimide solution under acidic conditions. The treated devices are rinsed in physiologic buffered saline solution and stored in a sodium azide preservative solution.

Using standard angiographic techniques, the above described devices when implanted in porcine coronary arteries and exposure to human blood produce an enhanced uptake and attachment of circulating endothelial progenitor cells on to the treated stent surface and accelerate their maturation into functional endothelium. The rapid establishment of functional endothelium is expected to decrease device thrombogenicity and modulate the extent of intimal hyperplasia.

### EXAMPLE 8

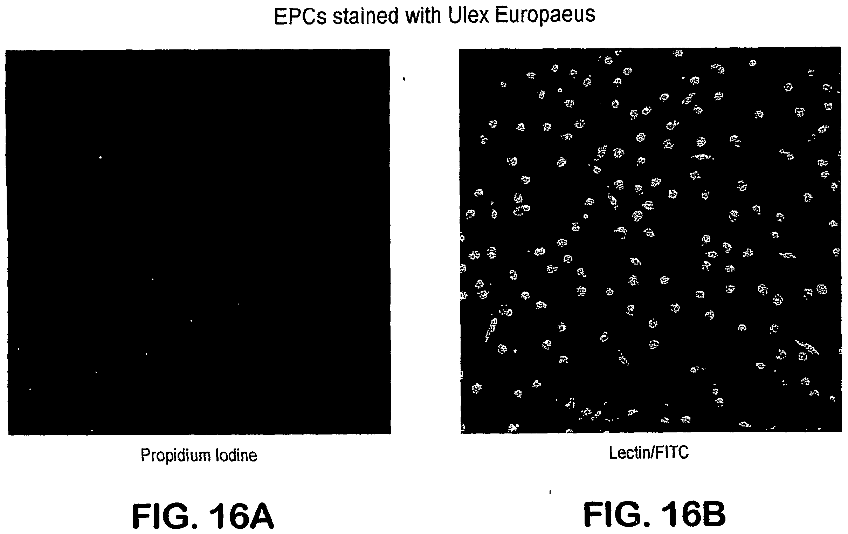

**Small Molecule Functionalization of a Stent: Progenitor** endothelial cells were isolated as described in Example 1. The cells were plated in fibronectin-coated slides and grown for 7 days in EBM-2 culture medium. Cells were fixed and stained with Propidium Iodine (PI) and a FITC-conjugated endothelial cell specific lectin. (Ulex Europaeus Uea 1) The results of these experiments are shown in FIGs. 16A and 16B. The figures show that progenitor endothelial cells are bound to the fibronectin-coated slides and that the cells express a ligand for the lectin on their surface.

## Claims

1. A medical device comprising a coating that promotes progenitor endothelial cells to adhere, grow and differentiate *in vivo* on the surface thereof when the device is implanted into a patient's organ or vessel, wherein the coating comprises: (a) at least one layer of a biocompatible matrix; (b) a therapeutically effective amount of at least one type of substance incorporated into the matrix and selected from the group consisting of antibodies, antibody fragments, combinations thereof and small molecules, wherein the at least one type of substance is directed against or interacts with a surface antigen on a progenitor endothelial cell and immobilizes the progenitor endothelial cell onto the surface of the medical device; and (c) at least one growth factor incorporated into the matrix.

2. The medical device of claim 1, wherein the medical device is selected from the group consisting of a stent, a stent graft, a synthetic vascular graft, a heart valve, a catheter, a vascular prosthetic filter, a pacemaker, a pacemaker lead, a defibrilator, a patent foramen ovale septal closure device, a vascular clip, a vascular aneurysm occluder, a hemodialysis graft, a hemodialysis catheter, an atrioventricular shunt, an aortic aneurysm graft device, a venous valve, a suture, a vascular anastomosis clip, an indwelling venous catheter, an indwelling arterial catheter, a vascular sheath and a drug delivery port.

3. The medical device of claim 2, wherein the medical device is a stent.

4. The medical device of claim 3, wherein the stent comprises a material selected from the group consisting of stainless steel, NiTi, MP35N, and chromium alloy.

5. The medical device of claim 3 or 4, wherein the stent further comprises a jacket, a covering or an encapsulation selected from the group consisting of a cross-linked PVA hydrogel, ePTFE, PTFE, porous HDPE, polyurethane, and polyethylene terephthalate.

6. The medical device of claim 2, wherein the medical device is a synthetic vascular graft and comprises a material selected from the group consisting of cross-linked polyvinyl alcohol, ePTFE, PTFE, porous HDPE, polyurethane, and polyethylene terephthalate.

7. The medical device of any of claims 1 to 6, wherein the biocompatible matrix comprises a synthetic material selected from the group consisting of a polyurethane, a segmented polyurethane-urea/heparin, a poly-L-lactic acid, cellulose ester, polyethylene glycol, polyvinyl acetate, dextran and gelatin; or wherein the biocompatible matrix comprises a naturally-occurring material selected from the group consisting of collagen, elastin, laminin, fibronectin, vitronectin, heparin, fibrin, cellulose and amorphous carbon; or wherein the biocompatible matrix comprises a fullerene ranging from about C₂₀ to about C₁₅₀ in the number of carbon atoms, wherein the fullerene is preferably arranged as a nanotube and/or wherein the fullerene is preferably C₆₀ or C₇₀.

8. The medical device of any of claims 1 to 7, wherein the antibody is selected from the group consisting of a monoclonal antibody, a polyclonal antibody, a chimeric antibody and a humanized antibody.

9. The medical device of any of claims 1 to 8, wherein the antibody or antibody fragment is covalently or noncovalently attached, or tethered covalently by a linker molecule to the outermost layer of the biocompatible matrix coating the medical device.

10. The medical device of any of claims 1 to 9, wherein the antibody or antibody fragment is specific for a human progenitor endothelial cell.

11. The medical device of any of claims 1 to 10, wherein the antibody or antibody fragment is directed against a progenitor endothelial cell surface antigen selected from the group consisting of CD133, CD34, CDw90, CD117, HLA-DR, VEGFR-1, VEGFR-2, Muc-18 (CD146), CD130, stem cell antigen (Sca-1), stem cell factor 1 (SCF/c-Kit ligand), Tie-2 and HAD-DR.

12. The medical device of any of claims 1 to 11, wherein the antibody is a monoclonal antibody which comprises Fab or F(ab')₂ fragments.

13. The medical device of any of claims 1 to 12, wherein the antibody fragment comprises small molecules of synthetic or natural origin.

14. The medical device of any of claims 1 to 13, wherein the growth factor is selected from the group consisting of vascular endothelial growth factor (VEGF), fibroblast growth factor (FGF)-3, FGF-4, FGF-5, FGF-6, FGF-7, FGF-8, FGF9, basic fibroblast growth factor, platelet-induced growth factor, transforming growth factor beta 1, acidic fibroblast growth factor, osteonectin, angiopoietin 1, angiopoietin 2, insulin-like growth factor, granulocyte-macrophage colony-stimulating factor, platelet-derived growth factor AA, platelet-derived growth factor BB, platelet-derived growth factor AB, endothelial PAS protein 1, thrombospondin, proliferin, Ephrin-A1, E-selectin, leptin, heparin, interleukin 8, thyroxine, and sphingosine 1-phosphate.

15. The medical device of claim 14, wherein the growth factor is a member of the VEGF family or Angiopoietin family.

16. The medical device of any of claims 1 to 15, wherein:
(a) the biocompatible matrix comprises a dextran, the at least one type of substance is a monoclonal antibody which binds CD34 cell surface antigen and the at least one growth factor is VEGF or Ang-2;
(b) the biocompatible matrix comprises a dextran, the at least one type of substance is a monoclonal antibody which binds CD133 cell surface antigen and the at least one growth factor is VEGF or Ang-2;
(c) the biocompatible matrix comprises a gelatin, the at least one type of substance is a monoclonal antibody which binds CD34 or CD 133 cell surface antigen and the at least one growth factor is VEGF or Ang-2;
(d) the biocompatible matrix comprises a gelatin or dextran, the at least one type of substance is a monoclonal antibody which binds Tie-2 cell surface antigen and the at least one growth factor is VEGF or Ang-2;
(e) the biocompatible matrix comprises a fullerene, the at least one type of substance is a monoclonal antibody which binds Tie-2 cell surface antigen and the at least one growth factor is VEGF or Ang-2; or
(f) the biocompatible matrix comprises a C₆₀ or C₇₀ fullerene, the at least one substance or fragment recognizes and binds the progenitor cell surface antigen CD34 or CD133, and the growth factor is VEGF or Ang-2.

17. A composition for coating a medical device, wherein the composition promotes progenitor endothelial cells to adhere, grow and differentiate *in vivo* on the coated surface of the device when the device is implanted into a patient's organ or vessel, the composition comprising (a) a biocompatible matrix, (b) a therapeutically effective amount of at least one type of substance to be incorporated into the matrix and selected from the group consisting of antibodies, antibody fragments, combinations thereof and small molecules, wherein the at least one type of substance is directed against or interacts with a surface antigen on a progenitor endothelial cell and immobilizes the progenitor endothelial cell onto the surface of the medical device; and (c) a therapeutically effective amount of at least one growth factor to be incorporated into the matrix and for stimulating the progenitor endothelial cells to differentiate into endothelial cells forming an endothelium on the surface of the medical device.

18. The composition of claim 17, wherein the biocompatible matrix comprises a synthetic material selected from the group consisting of a polyurethane, a segmented polyurethane-urea/heparin, a poly-L-lactic acid, cellulose ester, polyethylene glycol, polyvinyl acetate, dextran and gelatin; or wherein the biocompatible matrix comprises a naturally-occurring material selected from the group consisting of collagen, elastin, laminin, fibronectin, vitronectin, heparin, fibrin, cellulose and amorphous carbon; or wherein the biocompatible matrix comprises a fullerene ranging from about C₂₀ to about C₁₅₀.

19. The composition of claim 17 or 18, wherein the antibody or antibody fragment comprises a progenitor endothelial cell surface antigen selected from the group consisting of CD133, CD34, CDw90, CD117, HLA-DR, VEGFR-1, VEGFR-2, Muc-18 (CD146), CD130, stem cell antigen (Sca-1), stem cell factor 1 (SCF/c-Kit ligand), Tie-2 and HAD-DR.

20. The composition of any of claims 17 to 19, wherein the antibody is selected from the group consisting of a polyclonal, chimeric, humanized and monoclonal antibody, and wherein the monoclonal antibody comprises Fab or F(ab')₂ fragments.

21. The composition of any of claims 17 to 20, wherein the at least one growth factor is selected from the group consisting of vascular endothelial growth factor (VEGF), fibroblast growth factor (FGF)-3, FGF-4, FGF-5, FGF-6, FGF-7, FGF-8, FGF9, basic fibroblast growth factor, platelet-induced growth factor, transforming growth factor beta 1, acidic fibroblast growth factor, osteonectin, angiopoietin 1, angiopoietin 2, insulin-like growth factor, granulocyte-macrophage colony-stimulating factor, platelet-derived growth factor AA, platelet-derived growth factor BB, platelet-derived growth factor AB, endothelial PAS protein 1, trhombospondin, proliferin, Ephrin-A1, E-selectin, leptin, heparin, interleukin 8, thyroxine, and sphingosine 1-phosphate.

22. A method for coating a medical device comprising the steps of:
a. applying at least one layer of a biocompatible matrix to the surface of the medical device, wherein the biocompatible matrix comprises at least one component selected from the group consisting of a polyurethane, a segmented polyurethane-urea/heparin, a poly-L-lactic acid, a cellulose ester, a polyethylene glycol, a polyvinyl acetate, a dextran, gelatin, collagen, elastin, laminin, fibronectin, vitronectin, heparin, fibrin, cellulose and carbon and fullerene, and
b. applying to the biocompatible matrix, simultaneously or sequentially:
(i) a therapeutically effective amount of at least one type of substance to be incorporated into the matrix and selected from the group consisting of antibodies, antibody fragments, combinations thereof and small molecules, wherein the at least one type of substance is directed against or interacts with a surface antigen on a progenitor endothelial cell and immobilizes the progenitor endothelial cell onto the surface of the medical device, and
(ii) at least one growth factor to be incorporated into the matrix and which stimulates the progenitor endothelial cells to differentiate into endothelial cells forming an endothelium on the surface of the medical device.

23. The method of claim 22, wherein the medical device is selected from the group comprising a stent, a stent graft, a synthetic vascular graft, a heart valve, a catheter, a vascular prosthetic filter, a pacemaker, a pacemaker lead, a defibrilator, a patent foramen ovale septal closure device, a vascular clip, a vascular aneurysm occluder, a hemodialysis graft, a hemodialysis catheter, an atrioventricular shunt, an aortic aneurysm graft device, a venous valve, a suture, a vascular anastomosis clip, an indwelling venous catheter, an indwelling arterial catheter, a vascular sheath and a drug delivery port.

24. The method of claim 22 or 23, wherein the antibody is covalently or noncovalently attached on the biocompatible matrix coating the medical device.

25. The method of any of claims 22 to 24, wherein the fullerene is C₆₀ or C₇₀.

26. The method of any of claims 22 to 25, wherein the antibody or antibody fragment is directed against a progenitor endothelial cell surface antigen selected from the group consisting of CD133, CD34, CDw90, CD117, HLA-DR, VEGFR-1, VEGFR-2, Muc-18 (CD146), CD130, stem cell antigen (Sca-1), stem cell factor 1 (SCF/c-Kit ligand), Tie-2 and HAD-DR.

27. The method of any of claims 22 to 26, wherein the antibody is a monoclonal antibody and comprises a large or small molecule of the antibody.

28. The method of any of claims 22 to 27, wherein the at least one growth factor is selected from the group consisting of vascular endothelial growth factor (VEGF), fibroblast growth factor (FGF)-3, FGF-4, FGF-5, FGF-6, FGF-7, FGF-8, FGF9, basic fibroblast growth factor, platelet-induced growth factor, transforming growth factor beta 1, acidic fibroblast growth factor, osteonectin, angiopoietin 1, angiopoietin 2, insulin-like growth factor, granulocyte-macrophage colony-stimulating factor, platelet-derived growth factor AA, platelet-derived growth factor BB, platelet-derived growth factor AB, endothelial PAS protein 1, trhombospondin, proliferin, Ephrin-A1, E-selectin, leptin, heparin, interleukin 8, thyroxine, and sphingosine 1-phosphate.

29. The medical device of any of claims 1 to 7, wherein the small molecule is selected from the group consisting of a naturally occurring peptide, a synthetic peptide, a glycopeptide, a lipopeptide, a lipid, a saccharide, an organic molecule, an inorganic molecule, and a nucleic acid.

30. The medical device of any of claims 1 to 7 and 29, wherein the small molecule is covalently or noncovalently attached to the surface of the matrix, or tethered covalently by a linker molecule to the outermost layer of the matrix coating the medical device.

31. The medical device of any of claims 1 to 7, 29 and 30, wherein the small molecule is specific for a human progenitor endothelial cell.

32. The medical device of any of claims 1 to 7 and 29 to 31, wherein the small molecule is a ligand to a progenitor endothelial cell surface antigen selected from the group consisting of CD133, CD34, CDw90, CD117, HLA-DR, VEGFR-1, VEGFR-2, Muc-18 (CD146), CD130, stem cell antigen (Sca-1) stem cell factor 1 (SCF/c-Kit ligand), Tie-2 and HAD-DR.

## Patentansprüche

1. Medizinische Vorrichtung, umfassend eine Beschichtung, die endotheliale Vorläuferzellen dabei unterstützt, *in vivo* an ihrer Oberfläche anzuhaften, zu wachsen und sich zu differenzieren, wenn die Vorrichtung in ein Organ oder Gefäß eines Patienten implantiert wird, wobei die Beschichtung Folgendes umfasst: (a) wenigstens eine Schicht aus einer biokompatiblen Matrix; (b) eine therapeutisch wirksame Menge von wenigstens einem Substanztyp, der in die Matrix integriert ist und aus der Gruppe ausgewählt ist, die aus Antikörpern, Antikörperfragmenten, Kombinationen davon und kleinen Molekülen besteht, wobei der wenigstens eine Substanztyp gegen ein Oberflächenantigen an einer endothelialen Vorläuferzelle gerichtet ist oder mit diesem interagiert und die endotheliale Vorläuferzelle auf der Oberfläche der medizinischen Vorrichtung immobilisiert; und (c) wenigstens einen Wachstumsfaktor, der in die Matrix integriert ist.

2. Medizinische Vorrichtung nach Anspruch 1, wobei die medizinische Vorrichtung aus der Gruppe ausgewählt ist, die aus einem Stent, einem Stentimplantat, einem synthetischen Gefäßimplantat, einer Herzklappe, einem Katheter, einem Gefäßprothesenfilter, einem Herzschrittmacher, einer Herzschrittmachersonde, einem Defibrillator, einer Vorrichtung zum Verschluss eines persistierenden Foramen ovale im Septum, einer Gefäßklemme, einer Vorrichtung zum Verschluss eines Gefäßaneurysmas, einem Hämodialyse-Implantat, einem Hämodialyse-Katheter, einem atrioventrikulären Shunt, einer Implantatvorrichtung für ein Aortenaneurysma, einer Venenklappe, einer Naht, einer Gefäßanastomosenklemme, einem Venenverweilkatheter, einem Arterienverweilkatheter, einer Gefäßstütze und einem Arzneimittelabgabezugang besteht.

3. Medizinische Vorrichtung nach Anspruch 2, wobei die medizinische Vorrichtung ein Stent ist.

4. Medizinische Vorrichtung nach Anspruch 3, wobei der Stent ein Material umfasst, das aus der Gruppe ausgewählt ist, die aus rostfreiem Stahl, NiTi, MP35N und Chromlegierung besteht.

5. Medizinische Vorrichtung nach Anspruch 3 oder 4, wobei der Stent ferner eine Hülle, eine Beschichtung oder eine Verkapselung umfasst, die aus der Gruppe ausgewählt ist, die aus einem Hydrogel aus vernetztem PVA, ePTFE, PTFE, porösem HDPE, Polyurethan und Polyethylenterephthalat besteht.

6. Medizinische Vorrichtung nach Anspruch 2, wobei die medizinische Vorrichtung ein synthetisches Gefäßimplantat ist und ein Material umfasst, das aus der Gruppe ausgewählt ist, die aus vernetztem Polyvinylalkohol, ePTFE, PTFE, porösem HDPE, Polyurethan und Polyethylenterephthalat besteht.

7. Medizinische Vorrichtung nach einem der Ansprüche 1 bis 6, wobei die biokompatible Matrix ein synthetisches Material umfasst, das aus der Gruppe ausgewählt ist, die aus einem Polyurethan, einem segmentierten Polyurethan-Harnstoff/Heparin, einer Poly-L-Milchsäure, Celluloseester, Polyethylenglycol, Polyvinylacetat, Dextran und Gelatine besteht; oder wobei die biokompatible Matrix ein natürlich vorkommendes Material umfasst, das aus der Gruppe ausgewählt ist, die aus Collagen, Elastin, Laminin, Fibronectin, Vitronectin, Heparin, Fibrin, Cellulose und amorphem Kohlenstoff besteht; oder wobei die biokompatible Matrix ein Fulleren umfasst, dessen Anzahl an Kohlenstoffatomen im Bereich von etwa C₂₀ bis etwa C₁₅₀ liegt, wobei das Fulleren vorzugsweise als Nanoröhre angeordnet ist und/oder wobei das Fulleren vorzugsweise C₆₀ oder C₇₀ ist.

8. Medizinische Vorrichtung nach einem der Ansprüche 1 bis 7, wobei der Antikörper aus der Gruppe ausgewählt ist, die aus einem monoklonalen Antikörper, einem polyklonalen Antikörper, einem chimären Antikörper und einem humanisierten Antikörper besteht.

9. Medizinische Vorrichtung nach einem der Ansprüche 1 bis 8, wobei der Antikörper oder das Antikörperfragment kovalent oder nicht kovalent an die äußerste Schicht der biokompatiblen Matrix, welche die medizinische Vorrichtung bedeckt, geknüpft ist oder kovalent durch ein Linker-Molekül damit verbunden ist.

10. Medizinische Vorrichtung nach einem der Ansprüche 1 bis 9, wobei der Antikörper oder das Antikörperfragment spezifisch für eine menschliche endotheliale Vorläuferzelle ist.

11. Medizinische Vorrichtung nach einem der Ansprüche 1 bis 10, wobei der Antikörper oder das Antikörperfragment gegen ein Oberflächenantigen einer endothelialen Vorläuferzelle gerichtet ist, das aus der Gruppe ausgewählt ist, die aus CD133, CD34, CDw90, CD117, HLA-DR, VEGFR-1, VEGFR-2, Muc-18 (CD146), CD130, dem Stammzellantigen (Sca-1), dem Stammzellfaktor 1 (SCF/c-Kit-Ligand), Tie-2 und HAD-DR besteht.

12. Medizinische Vorrichtung nach einem der Ansprüche 1 bis 11, wobei der Antikörper ein monoklonaler Antikörper ist, der Fab- oder F(ab')₂-Fragmente umfasst.

13. Medizinische Vorrichtung nach einem der Ansprüche 1 bis 12, wobei das Antikörperfragment kleine Moleküle synthetischen oder natürlichen Ursprungs umfasst.

14. Medizinische Vorrichtung nach einem der Ansprüche 1 bis 13, wobei der Wachstumsfaktor aus der Gruppe ausgewählt ist, die aus dem Gefäßendothel-Wachstumsfaktor (VEGF), dem Fibroblasten-Wachstumsfaktor (FGF)-3, FGF-4, FGF-5, FGF-6, FGF-7, FGF-8, FGF9, dem basischen Fibroblasten-Wachstumsfaktor, dem Platelet-Induced Growth Factor, dem transformierenden Wachstumsfaktor Beta 1, dem sauren Fibroblasten-Wachstumsfaktor, Osteonectin, Angiopoietin 1, Angiopoietin 2, dem insulinähnlichen Wachstumsfaktor, dem Granulozyten-Makrophagen-Kolonie-stimulierenden Faktor, dem Platelet-Derived Growth Factor AA, dem Platelet-Derived Growth Factor BB, dem Platelet-Derived Growth Factor AB, dem endothelialen PAS-Protein 1, Thombospondin, Proliferin, Ephrin-A1, E-Selectin, Leptin, Heparin, Interleukin 8, Thyroxin und Sphingosin-1-phosphat besteht.

15. Medizinische Vorrichtung nach Anspruch 14, wobei der Wachstumfaktor ein Mitglied der VEGF-Familie oder der Angiopoietin-Familie ist.

16. Medizinische Vorrichtung nach einem der Ansprüche 1 bis 15, wobei:
(a) die biokompatible Matrix ein Dextran umfasst, der wenigstens eine Substanztyp ein monoklonaler Antikörper ist, der das Zelloberflächenantigen CD34 bindet und der wenigstens eine Wachstumsfaktor VEGF oder Ang-2 ist;
(b) die biokompatible Matrix ein Dextran umfasst, der wenigstens eine Substanztyp ein monoklonaler Antikörper ist, der das Zelloberflächenantigen CD133 bindet und der wenigstens eine Wachstumsfaktor VEGF oder Ang-2 ist;
(c) die biokompatible Matrix eine Gelatine umfasst, der wenigstens eine Substanztyp ein monoklonaler Antikörper ist, der das Zelloberflächenantigen CD34- oder CD133 bindet und der wenigstens eine Wachstumsfaktor VEGF oder Ang-2 ist;
(d) die biokompatible Matrix eine Gelatine oder ein Dextran umfasst, der wenigstens eine Substanztyp ein monoklonaler Antikörper ist, der das Zelloberflächenantigen Tie-2 bindet und der wenigstens eine Wachstumsfaktor VEGF oder Ang-2 ist;
(e) die biokompatible Matrix ein Fulleren umfasst, der wenigstens eine Substanztyp ein monoklonaler Antikörper ist, der das Zelloberflächenantigen Tie-2 bindet und der wenigstens eine Wachstumsfaktor VEGF oder Ang-2 ist; oder
(f) die biokompatible Matrix ein C₆₀- oder C₇₀-Fulleren umfasst, die wenigstens eine Substanz oder das wenigstens eine Fragment das Vorläuferzelloberflächenantigen CD34 oder CD133 erkennt und bindet und der Wachstumsfaktor VEGF oder Ang-2 ist.

17. Zusammensetzung zur Beschichtung einer medizinischen Vorrichtung, wobei die Zusammensetzung endotheliale Vorläuferzellen dabei unterstützt, *in vivo* an der beschichteten Oberfläche der Vorrichtung anzuhaften, zu wachsen und sich zu differenzieren, wenn die Vorrichtung in ein Organ oder Gefäß eines Patienten implantiert wird, wobei die Beschichtung Folgendes umfasst: (a) eine biokompatible Matrix; (b) eine therapeutisch wirksame Menge von wenigstens einem Substanztyp, der in die Matrix integriert werden soll und aus der Gruppe ausgewählt ist, die aus Antikörpern, Antikörperfragmenten, Kombinationen davon und kleinen Molekülen besteht, wobei der wenigstens eine Substanztyp gegen ein Oberflächenantigen an einer endothelialen Vorläuferzelle gerichtet ist oder mit diesem interagiert und die endotheliale Vorläuferzelle auf der Oberfläche der medizinischen Vorrichtung immobilisiert; und (c) eine therapeutisch wirksame Menge von wenigstens einem Wachstumsfaktor, der in die Matrix integriert werden soll und dazu dient, die endothelialen Vorläuferzellen derart zu stimulieren, dass sich diese in Endothelzellen differenzieren, die ein Endothel an der Oberfläche der medizinischen Vorrichtung bilden.

18. Zusammensetzung nach Anspruch 17, wobei die biokompatible Matrix ein synthetisches Material umfasst, das aus der Gruppe ausgewählt ist, die aus einem Polyurethan, einem segmentierten Polyurethan-Harnstoff/Heparin, einer Poly-L-Milchsäure, Celluloseester, Polyethylenglycol, Polyvinylacetat, Dextran und Gelatine besteht; oder wobei die biokompatible Matrix ein natürlich vorkommendes Material umfasst, das aus der Gruppe ausgewählt ist, die aus Collagen, Elastin, Laminin, Fibronectin, Vitronectin, Heparin, Fibrin, Cellulose und amorphem Kohlenstoff besteht; oder wobei die biokompatible Matrix ein Fulleren im Bereich von etwa C₂₀ bis etwa C₁₅₀ umfasst.

19. Zusammensetzung nach Anspruch 17 oder 18, wobei der Antikörper oder das Antikörperfragment ein Oberflächenantigen einer endothelialen Vorläuferzelle umfasst, das aus der Gruppe ausgewählt ist, die aus CD133, CD34, CDw90, CD117, HLA-DR, VEGFR-1, VEGFR-2, Muc-18 (CD146), CD130, dem Stammzellantigen (Sca-1), dem Stammzellfaktor 1 (SCF/c-Kit-Ligand), Tie-2 und HAD-DR besteht.

20. Zusammensetzung nach einem der Ansprüche 17 bis 19, wobei der Antikörper aus der Gruppe ausgewählt ist, die aus einem polyklonalen, einem chimären, einem humanisierten und einem monoklonalen Antikörper besteht und wobei der monoklonale Antikörper Fab- oder F(ab')₂-Fragmente umfasst.

21. Zusammensetzung nach einem der Ansprüche 17 bis 20, wobei der wenigstens eine Wachstumsfaktor aus der Gruppe ausgewählt ist, die aus dem Gefäßendothel-Wachstumfaktor (VEGF), dem Fibroblasten-Wachstumsfaktor (FGF)-3, FGF-4, FGF-5, FGF-6, FGF-7, FGF-8, FGF9, dem basischen Fibroblasten-Wachstumsfaktor, dem Platelet-Induced Growth Factor, dem transformierenden Wachstumsfaktor Beta 1, dem sauren Fibroblasten-Wachstumsfaktor, Osteonectin, Angiopoietin 1, Angiopoietin 2, dem insulinähnlichen Wachstumsfaktor, dem Granulozyten-Makrophagen-Kolonie-stimulierenden Faktor, dem Platelet-Derived Growth Factor AA, dem Platelet-Derived Growth Factor BB, dem Platelet-Derived Growth Factor AB, dem endothelialen PAS-Protein 1, Thombospondin, Proliferin, Ephrin-A1, E-Selectin, Leptin, Heparin, Interleukin 8, Thyroxin und Sphingosin-1-phosphat besteht.

22. Verfahren zum Beschichten einer medizinischen Vorrichtung, umfassend die folgenden Schritte:
a. Anbringen von wenigstens einer Schicht aus einer biokompatiblen Matrix an der Oberfläche der medizinischen Vorrichtung, wobei die biokompatible Matrix wenigstens eine Komponente umfasst, die aus der Gruppe ausgewählt ist, die aus einem Polyurethan, einem segmentierten Polyurethan-Harnstoff/Heparin, einer Poly-L-Milchsäure, einem Celluloseester, einem Polyethylenglycol, einem Polyvinylacetat, einem Dextran, Gelatine, Collagen, Elastin, Laminin, Fibronectin, Vitronectin, Heparin, Fibrin, Cellulose und Kohlenstoff und Fulleren besteht;
(b) gleichzeitig oder nacheinander erfolgendes Anbringen von Folgendem an der biokompatiblen Matrix:
(i) einer therapeutisch wirksamen Menge von wenigstens einem Substanztyp, der in die Matrix integriert werden soll und aus der Gruppe ausgewählt ist, die aus Antikörpern, Antikörperfragmenten, Kombinationen davon und kleinen Molekülen besteht, wobei der wenigstens eine Substanztyp gegen ein Oberflächenantigen an einer endothelialen Vorläuferzelle gerichtet ist oder mit diesem interagiert und die endotheliale Vorläuferzelle auf der Oberfläche der medizinischen Vorrichtung immobilisiert; und
(ii) wenigstens einem Wachstumsfaktor, der in die Matrix integriert werden soll und die endothelialen Vorläuferzellen derart stimuliert, dass sich diese in Endothelzellen differenzieren, die ein Endothel an der Oberfläche der medizinischen Vorrichtung bilden.

23. Verfahren nach Anspruch 22, wobei die medizinische Vorrichtung aus der Gruppe ausgewählt ist, die aus einem Stent, einem Stentimplantat, einem synthetischen Gefäßimplantat, einer Herzklappe, einem Katheter, einem Gefäßprothesenfilter, einem Herzschrittmacher, einer Herzschrittmachersonde, einem Defibrillator, einer Vorrichtung zum Verschluss eines persistierenden Foramen ovale im Septum, einer Gefäßklemme, einer Vorrichtung zum Verschluss eines Gefäßaneurysmas, einem Hämodialyse-Implantat, einem Hämodialyse-Katheter, einem atrioventrikulären Shunt, einer Implantatvorrichtung für ein Aortenaneurysma, einer Venenklappe, einer Naht, einer Gefäßanastomosenklemme, einem Venenverweilkatheter, einem Arterienverweilkatheter, einer Gefäßstütze und einem Arzneimittelabgabezugang besteht.

24. Verfahren nach Anspruch 22 oder 23, wobei der Antikörper kovalent oder nicht kovalent an die biokompatible Matrix geknüpft ist, welche die medizinische Vorrichtung bedeckt.

25. Verfahren nach einem der Ansprüche 22 bis 24, wobei das Fulleren C₆₀ oder C₇₀ ist.

26. Verfahren nach einem der Ansprüche 22 bis 25, wobei der Antikörper oder das Antikörperfragment gegen ein Oberflächenantigen einer endothelialen Vorläuferzelle gerichtet ist, das aus der Gruppe ausgewählt ist, die aus CD133, CD34, CDw90, CD117, HLA-DR, VEGFR-1, VEGFR-2, Muc-18 (CD146), CD130, dem Stammzellantigen (Sca-1), dem Stammzellfaktor 1 (SCF/c-Kit-Ligand), Tie-2 und HAD-DR besteht.

27. Verfahren nach einem der Ansprüche 22 bis 26, wobei der Antikörper ein monoklonaler Antikörper ist und ein großes oder kleines Molekül des Antikörpers umfasst.

28. Verfahren nach einem der Ansprüche 22 bis 27, wobei der wenigstens eine Wachstumsfaktor aus der Gruppe ausgewählt ist, die aus dem Gefäßendothel-Wachstumsfaktor (VEGF), dem Fibroblasten-Wachstumsfaktor (FGF)-3, FGF-4, FGF-5, FGF-6, FGF-7, FGF-8, FGF9, dem basischen Fibroblasten-Wachstumsfaktor, dem Platelet-Induced Growth Factor, dem transformierenden Wachstumsfaktor Beta 1, dem sauren Fibroblasten-Wachstumsfaktor, Osteonectin, Angiopoietin 1, Angiopoietin 2, dem insulinähnlichen Wachstumsfaktor, dem Granulozyten-Makrophagen-Kolonie-stimulierenden Faktor, dem Platelet-Derived Growth Factor AA, dem Platelet-Derived Growth Factor BB, dem Platelet-Derived Growth Factor AB, dem endothelialen PAS-Protein 1, Thombospondin, Proliferin, Ephrin-A1, E-Selectin, Leptin, Heparin, Interleukin 8, Thyroxin und Sphingosin-1-phosphat besteht.

29. Medizinische Vorrichtung nach einem der Ansprüche 1 bis 7, wobei das kleine Molekül aus der Gruppe ausgewählt ist, die aus einem natürlich vorkommenden Peptid, einem synthetischen Peptid, einem Glycopeptid, einem Lipopeptid, einem Lipid, einem Saccharid, einem organischen Molekül, einem anorganischen Molekül und einer Nukleinsäure besteht.

30. Medizinische Vorrichtung nach einem der Ansprüche 1 bis 7 und 29, wobei das kleine Molekül kovalent oder nicht kovalent an die Oberfläche der Matrix geknüpft ist oder kovalent durch ein Linker-Molekül mit der äußersten Schicht der Matrix verbunden ist, welche die medizinische Vorrichtung bedeckt.

31. Medizinische Vorrichtung nach einem der Ansprüche 1 bis 7, 29 und 30, wobei das kleine Molekül spezifisch für eine menschliche endotheliale Vorläuferzelle ist.

32. Medizinische Vorrichtung nach einem der Ansprüche 1 bis 7 und 29 bis 31, wobei das kleine Molekül ein Ligand an ein Oberflächenantigen einer endothelialen Vorläuferzelle ist, das aus der Gruppe ausgewählt ist, die aus CD133, CD34, CDw90, CD117, HLA-DR, VEGFR-1, VEGFR-2, Muc-18 (CD146), CD130, dem Stammzellantigen (Sca-1), dem Stammzellfaktor 1 (SCF/c-Kit-Ligand), Tie-2 und HAD-DR besteht.

## Revendications

1. Un dispositif médical comprenant un revêtement qui permet aux cellules endothéliales progénitrices d'adhérer, de se développer et de se différencier *in vivo* sur sa surface lorsque le dispositif est implanté dans l'organe ou le vaisseau d'un patient, dans lequel le revêtement comprend : (a) au moins une couche d'une matrice biocompatible ; (b) une quantité efficace d'un point de vue thérapeutique d'au moins un type de substance incorporée dans la matrice et sélectionnée dans le groupe constitué d'anticorps, de fragments d'anticorps, d'une combinaison de ceux-ci et de petites molécules, dans lequel la substance d'au moins un type est dirigée vers ou est en contact avec un antigène de surface sur une cellule endothéliale progénitrice et immobilise la cellule endothéliale progénitrice sur la surface du dispositif médical ; et (c) au moins un facteur de croissance incorporé dans la matrice.

2. Le dispositif médical selon la revendication 1, ledit dispositif médical étant sélectionné dans le groupe constitué d'un stent, d'une endoprothèse, d'un greffon vasculaire synthétique, d'une valve cardiaque, d'un cathéter, d'un filtre vasculaire prothétique, d'un *pacemaker,* d'une sonde de *pacemaker,* d'un défibrillateur, d'un dispositif de fermeture de septum pour foramen ovale perméable, d'une agrafe vasculaire, d'un dispositif d'occlusion d'anévrisme vasculaire, d'un greffon pour hémodialyse, d'un cathéter pour hémodialyse, d'un shunt auriculoventriculaire, d'un dispositif de greffon pour anévrisme aortique, d'une valvule veineuse, d'une suture, d'une agrafe vasculaire pour anastomose, d'une sonde veineuse, d'une sonde artérielle, d'une gaine vasculaire et d'un orifice d'administration de médicaments.

3. Le dispositif médical selon la revendication 2, ledit dispositif médical étant un stent.

4. Le dispositif médical selon la revendication 3, le stent comprenant un matériau sélectionné à partir du groupe constitué d'acier, de NiTi, de MP35N et d'alliage de chrome.

5. Le dispositif médical selon les revendications 3 ou 4, le stent comprenant également une enveloppe, un habillage ou un enrobage sélectionné à partir du groupe constitué d'hydrogel PVA réticulé, d'ePTFE, de PTFE, d'HDPE poreux, de polyuréthane et de téréphtalate de polyéthylène.

6. Le dispositif médical selon la revendication 2, ledit dispositif médical étant un greffon vasculaire synthétique et comprend un matériau sélectionné à partir du groupe constitué d'alcool polyvinylique réticulé, d'ePTFE, de PTFE, d'HDPE poreux, de polyuréthane et de téréphtalate de polyéthylène.

7. Le dispositif médical selon l'une quelconque des revendications 1 à 6, dans lequel la matrice biocompatible comprend un matériau synthétique sélectionné à partir du groupe constitué d'un polyuréthane, d'un mélange de polyuréthane-urée/héparine segmenté, d'un acide poly-L-lactique, d'ester de cellulose, de polyéthylène glycol, d'acétate de polyvinyle, de dextrane et de gélatine ; ou dans lequel la matrice biocompatible comprend un matériau produit naturellement sélectionné à partir du groupe constitué de collagène, d'élastine, de laminine, de fibronectine, de vitronectine, d'héparine, de fibrine, de cellulose et de carbone amorphe ; ou dans lequel la matrice biocompatible comprend un fullerène allant d'environ C₂₀ à environ C₁₅₀ en termes de nombre d'atomes de carbone, dans lequel le fullerène est de préférence disposé sous forme de nanotube et/ou dans lequel le fullerène est de préférence du C₆₀ ou du C₇₀.

8. Le dispositif médical selon l'une quelconque des revendications 1 à 7, dans lequel l'anticorps est sélectionné à partir du groupe constitué d'un anticorps monoclonal, d'un anticorps polyclonal, d'un anticorps chimérique et d'un anticorps humanisé.

9. Le dispositif médical selon l'une quelconque des revendications 1 à 8, dans lequel l'anticorps ou le fragment d'anticorps est lié de façon covalente ou non covalente, ou accroché de façon covalente par une molécule de liaison à la couche la plus externe de la matrice biocompatible recouvrant le dispositif médical.

10. Le dispositif médical selon l'une quelconque des revendications 1 à 9, dans lequel l'anticorps ou le fragment d'anticorps est spécifique à une cellule endothéliale progénitrice humaine.

11. Le dispositif médical selon l'une quelconque des revendications 1 à 10, dans lequel l'anticorps ou le fragment d'anticorps est dirigé vers un antigène de surface de cellule endothéliale progénitrice sélectionné à partir du groupe constitué du CD133, du CD34, du CDw90, du CD117, du HLA-DR, du VEGFR-1, du VEGFR-2, du Muc-18 (CD146), du CD130, de l'antigène de cellule souche (Sca-1), du facteur 1 de croissance de cellules souches (SCF/ligand de c-kit), du Tie-2 et du HAD-DR.

12. Le dispositif médical selon l'une quelconque des revendications 1 à 11, dans lequel l'anticorps est un anticorps monoclonal qui comprend des fragments Fab ou F(ab')2.

13. Le dispositif médical selon l'une quelconque des revendications 1 à 12, dans lequel le fragment d'anticorps comprend de petites molécules d'origine synthétique ou naturelle.

14. Le dispositif médical selon l'une quelconque des revendications 1 à 13, dans lequel le facteur de croissance est sélectionné à partir du groupe constitué du facteur de croissance de l'endothélium vasculaire (VEGF), du facteur-3 de croissance fibroblastique (FGF)-3, du FGF-4, du FGF-5, du FGF-6, du FGF-7, du FGF-8, du FGF-9, du facteur de croissance fibroblastique basique, du facteur de croissance dérivé des plaquettes, du facteur de croissance transformant bêta 1, du facteur de croissance fibroblastique acide, de l'ostéonectine, de l'angiopoiétine 1, de l'angiopoiétine 2, du facteur de croissance semblable à l'insuline, du facteur de stimulation des colonies de granulocytes et de macrophages, du facteur de croissance AA dérivé des plaquettes, du facteur de croissance BB dérivé des plaquettes, du facteur de croissance AB dérivé des plaquettes, de la protéine à domaine PAS endothelial 1, de la thrombospondine, de la proliférine, de l'éphrine-A1, de l'E-sélectine, de la leptine, de l'héparine, de l'interleukine 8, de la thyroxine et de la sphingosine-1-phosphate.

15. Le dispositif médical selon la revendication 14, dans lequel le facteur de croissance fait partie de la famille du VEFG ou de la famille de l'angiopoiétine.

16. Le dispositif médical selon l'une quelconque des revendications 1 à 15, dans lequel :
(a) la matrice biocompatible comprend un dextrane, la substance d'au moins un type est un anticorps monoclonal qui fixe l'antigène de surface de cellule CD34 et le facteur de croissance est le VEGF ou l'Ang-2 ;
(b) la matrice biocompatible comprend un dextrane, la substance d'au moins un type est un anticorps monoclonal qui fixe l'antigène de surface de cellule CD133 et le facteur de croissance est le VEGF ou l'Ang-2 ;
(c) la matrice biocompatible comprend une gélatine, la substance d'au moins un type est un anticorps monoclonal qui fixe l'antigène de surface de cellule CD34 ou CD133 et le facteur de croissance est le VEGF ou l'Ang-2 ;
(d) la matrice biocompatible comprend une gélatine ou un dextrane, la substance d'au moins un type est un anticorps monoclonal qui fixe l'antigène de surface de cellule Tie-2 et le facteur de croissance est le VEGF ou l'Ang-2 ;
(e) la matrice biocompatible comprend un fullerène, la substance d'au moins un type est un anticorps monoclonal qui fixe l'antigène de surface de cellule Tie-2 et le facteur de croissance est le VEGF ou l'Ang-2 ;
(f) la matrice biocompatible comprend un fullerène C₆₀ ou C₇₀, la substance ou le fragment reconnaît et fixe l'antigène de surface de cellule progénitrice CD34 ou CD 133, et le facteur de croissance est le VEGF ou le Ang-2.

17. Une composition destinée à recouvrir un dispositif médical, ladite composition permettant aux cellules endothéliales progénitrices d'adhérer, de se développer et de se différencier *in vivo* sur la surface recouverte du dispositif lorsque celui-ci est implanté dans l'organe ou le vaisseau d'un patient, la composition comprenant : (a) une matrice biocompatible, (b) une quantité efficace d'un point de vue thérapeutique d'au moins un type de substance devant être incorporée dans la matrice et sélectionnée à partir du groupe constitué d'anticorps, de fragments d'anticorps, d'une combinaison de ceux-ci et de petites molécules, dans lequel la substance d'au moins un type est dirigée vers ou est en contact avec un antigène de surface sur une cellule endothéliale progénitrice et immobilise la cellule endothéliale progénitrice sur la surface du dispositif médical ; et (c) une quantité efficace d'un point de vue thérapeutique d'au moins un facteur de croissance devant être incorporé dans la matrice et pour stimuler les cellules endothéliales progénitrices afin qu'elles se différencient en cellules endothéliales formant un endothélium sur la surface du dispositif médical.

18. La composition selon la revendication 17, dans laquelle la matrice biocompatible comprend un matériau synthétique sélectionné à partir du groupe constitué d'un polyuréthane, d'un mélange de polyuréthane-urée/héparine segmenté, d'un acide poly-L-lactique, d'ester de cellulose, de polyéthylène glycol, d'acétate de polyvinyle, de dextrane et de gélatine ; ou dans laquelle la matrice biocompatible comprend un matériau produit naturellement sélectionné à partir du groupe constitué de collagène, d'élastine, de laminine, de fibronectine, de vitronectine, d'héparine, de fibrine, de cellulose et de carbone amorphe ; ou dans laquelle la matrice biocompatible comprend un fullerène allant d'environ C₂₀ à environ C₁₅₀.

19. La composition selon les revendications 17 ou 18, dans laquelle l'anticorps ou le fragment d'anticorps comprend un antigène de surface de cellule endothéliale progénitrice sélectionné à partir du groupe constitué du CD133, du CD34, du CDw90, du CD117, du HLA-DR, du VEGFR-1, du VEGFR-2, du Muc-18 (CD146), du CD130, de l'antigène de cellule souche (Sca-1), du facteur 1 de croissance de cellules souches (SCF/ligand de c-kit), du Tie-2 et du HAD-DR.

20. La composition selon l'une quelconque des revendications 17 à 19, dans laquelle l'anticorps est sélectionné à partit du groupe constitué d'un anticorps polyclonal, chimérique, humanisé et monoclonal, et dans laquelle l'anticorps monoclonal comprend des fragments Fab ou F(ab')2.

21. La composition selon l'une quelconque des revendications 17 à 20, dans laquelle le facteur de croissance est sélectionné à partir du groupe constitué du facteur de croissance de l'endothélium vasculaire (VEGF), du facteur-3 de croissance fibroblastique (FGF)-3, du FGF-4, du FGF-5, du FGF-6, du FGF-7, du FGF-8, du FGF-9, du facteur de croissance fibroblastique basique, du facteur de croissance dérivé des plaquettes, du facteur de croissance transformant bêta 1, du facteur de croissance fibroblastique acide, de l'ostéonectine, de l'angiopoiétine 1, de l'angiopoiétine 2, du facteur de croissance semblable à l'insuline, du facteur de stimulation des colonies de granulocytes et de macrophages, du facteur de croissance AA dérivé des plaquettes, du facteur de croissance BB dérivé des plaquettes, du facteur de croissance AB dérivé des plaquettes, de la protéine à domaine PAS endothelial 1, de la thrombospondine, de la proliférine, de l'éphrine-A1, de l'E-sélectine, de la leptine, de l'héparine, de l'interleukine 8, de la thyroxine et de la sphingosine-1-phosphate.

22. Une méthode de revêtement d'un dispositif médical comprenant les étapes suivantes :
(a) application d'au moins une couche d'une matrice biocompatible sur la surface du dispositif médical, la matrice biocompatible comprenant au moins une composante sélectionnée à partir du groupe constitué d'un polyuréthane, d'un mélange de polyuréthane-urée/héparine segmenté, d'un acide poly-L-lactique, d'un ester de cellulose, d'un polyéthylène glycol, d'un acétate de polyvinyle, d'un dextrane, de gélatine, de collagène, d'élastine, de laminine, de fibronectine, de vitronectine, d'héparine, de fibrine, de cellulose et de carbone et de fullerène, et
(b) application à la matrice biocompatible, simultanément ou successivement :
(i) d'une quantité efficace d'un point de vue thérapeutique d'au moins un type de substance devant être incorporée dans la matrice et sélectionnée à partir du groupe constitué d'anticorps, de fragments d'anticorps, d'une combinaison de ceux-ci et de petites molécules, la substance d'au moins un type étant dirigée vers ou est en contact avec un antigène de surface sur une cellule endothéliale progénitrice et immobilise la cellule endothéliale progénitrice sur la surface du dispositif médical, et
(ii) d'au moins un facteur de croissance devant être incorporé dans la matrice et qui stimule les cellules endothéliales progénitrices afin qu'elles se différencient en cellules endothéliales formant un endothélium sur la surface du dispositif médical.

23. La méthode selon la revendication 22, dans laquelle le dispositif médical est sélectionné dans le groupe comprenant un stent, une endoprothèse, un greffon vasculaire synthétique, une valve cardiaque, un cathéter, un filtre vasculaire prothétique, un *pacemaker,* une sonde de *pacemaker,* un défibrillateur, un dispositif de fermeture de septum pour foramen ovale perméable, une agrafe vasculaire, un dispositif d'occlusion d'anévrisme vasculaire, un greffon pour hémodialyse, un cathéter pour hémodialyse, un shunt auriculoventriculaire, un dispositif de greffon pour anévrisme aortique, une valvule veineuse, une suture, une agrafe vasculaire pour anastomose, une sonde veineuse, une sonde artérielle, une gaine vasculaire et un orifice d'administration de médicaments.

24. La méthode selon la revendication 22 ou 23, dans laquelle l'anticorps est lié de façon covalente ou non covalente à la matrice biocompatible recouvrant le dispositif médical.

25. La méthode selon l'une quelconque des revendications 22 à 24, dans laquelle le fullerène est du C₆₀ ou du C₇₀.

26. La méthode selon l'une quelconque des revendications 22 à 25, dans laquelle l'anticorps ou le fragment d'anticorps est dirigé vers un antigène de surface de cellule endothéliale progénitrice sélectionné à partir du groupe constitué du CD133, du CD34, du CDw90, du CD117, du HLA-DR, du VEGFR-1, du VEGFR-2, du Muc-18 (CD 146), du CD130, de l'antigène de cellule souche (Sca-1), du facteur 1 de croissance de cellules souches (SCF/ligand de c-kit), du Tie-2 et du HAD-DR.

27. La méthode selon l'une quelconque des revendications 22 à 26, dans laquelle l'anticorps est un anticorps monoclonal et comprend une grande ou une petite molécule de l'anticorps.

28. La méthode selon l'une quelconque des revendications 22 à 26, dans laquelle le facteur de croissance est sélectionné à partir du groupe constitué du facteur de croissance de l'endothélium vasculaire (VEGF), du facteur-3 de croissance fibroblastique (FGF)-3, du FGF-4, du FGF-5, du FGF-6, du FGF-7, du FGF-8, du FGF-9, du facteur de croissance fibroblastique basique, du facteur de croissance dérivé des plaquettes, du facteur de croissance transformant bêta 1, du facteur de croissance fibroblastique acide, de l'ostéonectine, de l'angiopoiétine 1, de l'angiopoiétine 2, du facteur de croissance semblable à l'insuline, du facteur de stimulation des colonies de granulocytes et de macrophages, du facteur de croissance AA dérivé des plaquettes, du facteur de croissance BB dérivé des plaquettes, du facteur de croissance AB dérivé des plaquettes, de la protéine à domaine PAS endothelial 1, de la thrombospondine, de la proliférine, de l'éphrine-A1, de l'E-sélectine, de la leptine, de l'héparine, de l'interleukine 8, de la thyroxine et de la sphingosine-1-phosphate.

29. Le dispositif médical selon l'une quelconque des revendications 1 à 7, dans lequel la petite molécule est sélectionnée à partir du groupe constitué d'un peptide produit naturellement, d'un peptide synthétique, d'un glycopeptide, d'un lipopeptide, d'un lipide, d'un saccharide, d'une molécule organique, d'une molécule inorganique et d'un acide nucléique.

30. Le dispositif médical selon l'une quelconque des revendications 1 à 7 et 29, dans lequel la petite molécule est liée de façon covalente ou non covalente à la surface de la matrice, ou accrochée de façon covalente par une molécule de liaison à la couche la plus externe de la matrice biocompatible recouvrant le dispositif médical.

31. Le dispositif médical selon l'une quelconque des revendications 1 à 7, 29 et 30, dans lequel la petite molécule est spécifique à une cellule endothéliale progénitrice humaine.

32. Le dispositif médical selon l'une quelconque des revendications 1 à 7 et 29 à 31, dans lequel la petite molécule est un ligand à un antigène de surface de cellule endothéliale progénitrice sélectionné à partir du groupe constitué du CD133, du CD34, du CDw90, du CD117, du HLA-DR, du VEGFR-1, du VEGFR-2, du Muc-18 (CD 146), du CD130, de l'antigène de cellule souche (Sca-1), du facteur 1 de croissance de cellules souches (SCF/ligand de c-kit), du Tie-2 et du HAD-DR.
